# EUROPÄISCHE PATENTSCHRIFT

(11) **EP 1 660 532 B1**
(45) Veröffentlichungstag und Bekanntmachung des Hinweises auf die Patenterteilung: **24.10.2012**
(21) Anmeldenummer: 04764346.5
(22) Anmeldetag: 20.08.2004
(51) Int. Cl.: C07K 14/745, C07K 19/00, A61K 38/36

(54) **FUSIONSPOLYPEPTIDE UND DEREN VERWENDUNG FÜR DIE ANTIVASKULÄRE TUMORTHERAPIE**
FUSION POLYPEPTIDES, AND USE THEREOF IN ANTIVASCULAR TUMOR THERAPY
POLYPEPTIDE DE FUSION ET SON UTILISATION EN THERAPIE DES TUMEURS ANTIVASCULAIRES

(30) Priorität: 22.08.2003 DE 10338733
(43) Veröffentlichungstag der Anmeldung: 31.05.2006
(73) Patentinhaber: Berdel, Wolfgang, 48149 Münster (DE); Oncoscience AG, 22880 Wedel (DE); Bizimis, Ari, E., 61462 Königstein (DE)
(72) Erfinder: BERDEL, Wolfgang, E., 48149 Münster (DE); MESTERS, Rolf, M., 48149 Münster (DE)
(74) Vertreter: UEXKÜLL & STOLBERG
(86) Internationale Anmeldenummer: PCT/EP2004/009364
(87) Internationale Veröffentlichungsnummer: WO 2005/021593

(56) Entgegenhaltungen:
- WO-A-03/035688
- HU PEISHENG ET AL: "Comparison of three different targeted Tissue Factor fusion proteins for inducing tumor vessel thrombosis." CANCER RESEARCH, Bd. 63, Nr. 16, 15. August 2003 (2003-08-15), Seiten 5046-5053, XP002316414 ISSN: 0008-5472 in der Anmeldung erwähnt
- RIPPMANN J F ET AL: "FUSION OF THE TISSUE FACTOR EXTRACELLULAR DOMAIN TO A TUMOUR STROMASPECIFIC SINGLE-CHAIN FRAGMENT VARIABLE ANTIBODY RESULTS IN AN ANTIGEN-SPECIFIC COAGULATION-PROMOTING MOLECULE" BIOCHEMICAL JOURNAL, THE BIOCHEMICAL SOCIETY, LONDON, GB, Bd. 349, Nr. 3, 1. August 2000 (2000-08-01), Seiten 805-812, XP001021508 ISSN: 0264-6021
- ARAP W ET AL: "CANCER TREATMENT BY TARGETED DRUG DELIVERY TO TUMOR VASCULATURE IN A MOUSE MODEL" SCIENCE, AMERICAN ASSOCIATION FOR THE ADVANCEMENT OF SCIENCE,, US, Bd. 279, 16. Januar 1998 (1998-01-16), Seiten 377-380, XP000857470 ISSN: 0036-8075 in der Anmeldung erwähnt

### SEQUENZPROTOKOLL

<110> Medizinische Klinik und Poliklinik A des Universitätsklinikums Münster
<120> Fusionspolypeptide für die antivaskuläre Tumortherapie
<130> P 51875
<160> 31
<170> PatentIn version 3.1
<210> 1
   <211> 263
   <212> PRT
   <213> Homo sapiens
<220>
   <221> Aminosäuresequenz von humanem TF
<400> 1
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
<210> 2
   <211> 2
   <212> PRT
   <213> Homo sapiens
<220>
   <221> Aminosäuresequenz von tTF₁₋₂₁₈
<400> 2
<210> 3
   <211> 224
   <212> PRT
   <213> Artificial
<220>
   <221> Aminosäuresequenz von tTF-GRGDSP
<400> 3
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Gly Arg Gly Asp Ser Asp
       210                 215                 220
<210> 4
   <211> 225
   <212> PRT
   <213> Artificial
<220>
   <221> Aminosäuresequenz von tTF-GNGRAHA
<400> 4
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Gly Asn Gly Arg Ala His
       210                 215                 220
   Ala
   225
<210> 5
   <211> 228
   <212> PRT
   <213> Artificial
<220>
   <221> Aminosäuresequenz von tTF-GALNGRSHAG
<400> 5
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Gly Ala Leu Asn Gly Arg
       210                 215                 220
   Ser His Ala Gly
   225
<210> 6
   <211> 225
   <212> PRT
   <213> Artificial
<220>
   <221> Aminosäuresequenz von tTF-GCNGRCG
<400> 6
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Gly Cys Asn Gly Arg Cys
       210                 215                 220
   Gly
   225
<210> 7
   <211> 232
   <212> PRT
   <213> Artificial
<220>
   <221> Aminosäuresequenz von tTF-GCNGRCVSGCAGRC
<400> 7
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Gly Cys Asn Gly Arg Cys
       210                 215                 220
   Val Ser Gly Cys Ala Gly Arg Cys
   225                 230
<210> 8
   <211> 228
   <212> PRT
   <213> Artificial
<220>
   <221> Aminosäuresequenz von tTF-GCVLNGRMEC
<400> 8
   Ser Gly Thr Thr Asn Thr Val Ala Ala Tyr Asn Leu Thr Trp Lys Ser
   1               5                   10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Gly Cys Val Leu Asn Gly
       210                 215                 220
   Arg Met Glu Cys
   225
<210> 9
   <211> 654
   <212> DNA
   <213> Artifical
<220>
   <221> Nukleotidsequenz von tTF₁₋₂₁₈
<400> 9
   tcaggcacta caaatactgt ggcagcatat aatttaactt ggaaatcaac taatttcaag      60
   acaattttgg agtgggaacc caaacccgtc aatcaagtct acactgttca aataagcact     120
   aagtcaggag attggaaaag caaatgcttt tacacaacag acacagagtg tgacctcacc     180
   gacgagattg tgaaggatgt gaagcagacg tacttggcac gggtcttctc ctacccggca     240
   gggaatgtgg agagcaccgg ttctgctggg gagcctctgt atgagaactc cccagagttc     300
   acaccttacc tggagacaaa cctcggacag ccaacaattc agagttttga acaggtggga     360
   acaaaagtga atgtgaccgt agaagatgaa cggactttag tcagaaggaa caacactttc     420
   ctaagcctcc gggatgtttt tggcaaggac ttaatttata cactttatta ttggaaatct     480
   tcaagttcag gaaagaaaac agccaaaaca aacactaatg agtttttgat tgatgtggat     540
   aaaggagaaa actactgttt cagtgttcaa gcagtgattc cctcccgaac agttaaccgg     600
   aagagtacag acagcccggt agagtgtatg ggccaggaga aaggggaatt caga           654
<210> 10
   <211> 672
   <212> DNA
   <213> Artificial
<220>
   <221> Nukleotidsequenz von tTF-GRGDSP
<400> 10
   tcaggcacta caaatactgt ggcagcatat aatttaactt ggaaatcaac taatttcaag      60
   acaattttgg agtgggaacc caaacccgtc aatcaagtct acactgttca aataagcact     120
   aagtcaggag attggaaaag caaatgcttt tacacaacag acacagagtg tgacctcacc     180
   gacgagattg tgaaggatgt gaagcagacg tacttggcac gggtcttctc ctacccggca     240
   gggaatgtgg agagcaccgg ttctgctggg gagcctctgt atgagaactc cccagagttc     300
   acaccttacc tggagacaaa cctcggacag ccaacaattc agagttttga acaggtggga     360
   acaaaagtga atgtgaccgt agaagatgaa cggactttag tcagaaggaa caacactttc     420
   ctaagcctcc gggatgtttt tggcaaggac ttaatttata cactttatta ttggaaatct     480
   tcaagttcag gaaagaaaac agccaaaaca aacactaatg agtttttgat tgatgtggat     540
   aaaggagaaa actactgttt cagtgttcaa gcagtgattc cctcccgaac agttaaccgg     600
   aagagtacag acagcccggt agagtgtatg ggccaggaga aaggggaatt cagaggaaga     660
   ggtgattctc ca                                                         672
<210> 11
   <211> 675
   <212> DNA
   <213> Artificial
<220>
   <221> Nukleotidsequenz von tTF-GNGRAHA
<400> 11
   tcaggcacta caaatactgt ggcagcatat aatttaactt ggaaatcaac taatttcaag      60
   acaattttgg agtgggaacc caaacccgtc aatcaagtct acactgttca aataagcact     120
   aagtcaggag attggaaaag caaatgcttt tacacaacag acacagagtg tgacctcacc     180
   gacgagattg tgaaggatgt gaagcagacg tacttggcac gggtcttctc ctacccggca     240
   gggaatgtgg agagcaccgg ttctgctggg gagcctctgt atgagaactc cccagagttc     300
   acaccttacc tggagacaaa cctcggacag ccaacaattc agagttttga acaggtggga     360
   acaaaagtga atgtgaccgt agaagatgaa cggactttag tcagaaggaa caacactttc     420
   ctaagcctcc gggatgtttt tggcaaggac ttaatttata cactttatta ttggaaatct     480
   tcaagttcag gaaagaaaac agccaaaaca aacactaatg agtttttgat tgatgtggat     540
   aaaggagaaa actactgttt cagtgttcaa gcagtgattc cctcccgaac agttaaccgg     600
   aagagtacag acagcccggt agagtgtatg ggccaggaga aaggggaatt cagaggtaac     660
   ggaagagcac atgca                                                      675
<210> 12
   <211> 684
   <212> DNA
   <213> Artificial
<220>
   <221> Nukleotidsequenz von tTF-GALNGRSHAG
<400> 12
   tcaggcacta caaatactgt ggcagcatat aatttaactt ggaaatcaac taatttcaag      60
   acaattttgg agtgggaacc caaacccgtc aatcaagtct acactgttca aataagcact     120
   aagtcaggag attggaaaag caaatgcttt tacacaacag acacagagtg tgacctcacc     180
   gacgagattg tgaaggatgt gaagcagacg tacttggcac gggtcttctc ctacccggca     240
   gggaatgtgg agagcaccgg ttctgctggg gagcctctgt atgagaactc cccagagttc     300
   acaccttacc tggagacaaa cctcggacag ccaacaattc agagttttga acaggtggga     360
   acaaaagtga atgtgaccgt agaagatgaa cggactttag tcagaaggaa caacactttc 420
   ctaagcctcc gggatgtttt tggcaaggac ttaatttata cactttatta ttggaaatct 480
   tcaagttcag gaaagaaaac agccaaaaca aacactaatg agtttttgat tgatgtggat 540
   aaaggagaaa actactgttt cagtgttcaa gcagtgattc cctcccgaac agttaaccgg 600
   aagagtacag acagcccggt agagtgtatg ggccaggaga aaggggaatt cagaggtgct 660
   ttaaatggaa gatctcacgc tggt 684
<210> 13
   <211> 675
   <212> DNA
   <213> Artificial
<220>
   <221> Nukleotidsequenz von tTF-GCNGRCG
<400> 13
   tcaggcacta caaatactgt ggcagcatat aatttaactt ggaaatcaac taatttcaag 60
   acaattttgg agtgggaacc caaacccgtc aatcaagtct acactgttca aataagcact 120
   aagtcaggag attggaaaag caaatgcttt tacacaacag acacagagtg tgacctcacc 180
   gacgagattg tgaaggatgt gaagcagacg tacttggcac gggtcttctc ctacccggca 240
   gggaatgtgg agagcaccgg ttctgctggg gagcctctgt atgagaactc cccagagttc 300
   acaccttacc tggagacaaa cctcggacag ccaacaattc agagttttga acaggtggga 360
   acaaaagtga atgtgaccgt agaagatgaa cggactttag tcagaaggaa caacactttc 420
   ctaagcctcc gggatgtttt tggcaaggac ttaatttata cactttatta ttggaaatct 480
   tcaagttcag gaaagaaaac agccaaaaca aacactaatg agtttttgat tgatgtggat 540
   aaaggagaaa actactgttt cagtgttcaa gcagtgattc cctcccgaac agttaaccgg 600
   aagagtacag acagcccggt agagtgtatg ggccaggaga aaggggaatt cagaggctgc 660
   aacggtagat gtggt 675
<210> 14
   <211> 696
   <212> DNA
   <213> Artificial
<220>
   <221> Nukleotidsequenz von tTF-GCNGRCVSGCAGRC
<400> 14
   tcaggcacta caaatactgt ggcagcatat aatttaactt ggaaatcaac taatttcaag 60
   acaattttgg agtgggaacc caaacccgtc aatcaagtct acactgttca aataagcact 120
   aagtcaggag attggaaaag caaatgcttt tacacaacag acacagagtg tgacctcacc 180
   gacgagattg tgaaggatgt gaagcagacg tacttggcac gggtcttctc ctacccggca 240
   gggaatgtgg agagcaccgg ttctgctggg gagcctctgt atgagaactc cccagagttc 300
   acaccttacc tggagacaaa cctcggacag ccaacaattc agagttttga acaggtggga 360
   acaaaagtga atgtgaccgt agaagatgaa cggactttag tcagaaggaa caacactttc 420
   ctaagcctcc gggatgtttt tggcaaggac ttaatttata cactttatta ttggaaatct 480
   tcaagttcag gaaagaaaac agccaaaaca aacactaatg agtttttgat tgatgtggat 540
   aaaggagaaa actactgttt cagtgttcaa gcagtgattc cctcccgaac agttaaccgg 600
   aagagtacag acagcccggt agagtgtatg ggccaggaga aaggggaatt cagaggttgt 660
   aatggaagat gtgtttctgg atgtgcagga cgatgt 696
<210> 15
   <211> 684
   <212> DNA
   <213> Artificial
<220>
   <221> Nukleotidsequenz von tTF-GCVLNGRMEC
<400> 15
   tcaggcacta caaatactgt ggcagcatat aatttaactt ggaaatcaac taatttcaag 60
   acaattttgg agtgggaacc caaacccgtc aatcaagtct acactgttca aataagcact 120
   aagtcaggag attggaaaag caaatgcttt tacacaacag acacagagtg tgacctcacc 180
   gacgagattg tgaaggatgt gaagcagacg tacttggcac gggtcttctc ctacccggca 240
   gggaatgtgg agagcaccgg ttctgctggg gagcctctgt atgagaactc cccagagttc 300
   acaccttacc tggagacaaa cctcggacag ccaacaattc agagttttga acaggtggga 360
   acaaaagtga atgtgaccgt agaagatgaa cggactttag tcagaaggaa caacactttc 420
   ctaagcctcc gggatgtttt tggcaaggac ttaatttata cactttatta ttggaaatct 480
   tcaagttcag gaaagaaaac agccaaaaca aacactaatg agtttttgat tgatgtggat 540
   aaaggagaaa actactgttt cagtgttcaa gcagtgattc cctcccgaac agttaaccgg 600
   aagagtacag acagcccggt agagtgtatg ggccaggaga aaggggaatt cagaggatgc 660
   gtcttaaatg gtaggatgga atgc 684
<210> 16
   <211> 45
   <212> DNA
   <213> Artifical
<220>
   <221> 5' Oligonukleotidprimer für die Herstellung von tTF₁₋₂₁₈
<400> 16
   catgccatgg gatcaggcac tacaaatact gtggcagcat ataat 45
<210> 17
   <211> 40
   <212> DNA
   <213> Artifical
<220>
   <221> 3' Oligonukleotidprimer für die Herstellung von tTF₁₋₂₁₈
<400> 17
   cgggatccta ttatctgaat tcccctttct cctggcccat 40
<210> 18
   <211> 45
   <212> DNA
   <213> Artificial
<220>
   <221> 5' Oligonukleotidprimer für die Herstellung von tTF-GRGDSP
<400> 18
   catgccatgg gatcaggcac tacaaatact gtggcagcat ataat 45
<210> 19
   <211> 43
   <212> DNA
   <213> Artificial
<220>
   <221> 3' Oligonukleotidprimer für die Herstellung von tTF-GRGDSP
<400> 19
   cgggatccta ttatggagaa tcacctcttc ctctgaattc ccc 43
<210> 20
   <211> 45
   <212> DNA
   <213> Artificial
<220>
   <221> 5' Oligonukleotidprimer für die Herstellung von tTF-GNGRAHA
<400> 20
   catgccatgg gatcaggcac tacaaatact gtggcagcat ataat 45
<210> 21
   <211> 46
   <212> DNA
   <213> Artificial
<220>
   <221> 3' Oligonukleotidprimer für die Herstellung von tTF-GNGRAHA
<400> 21
   cgggatccta ttatgcatgt gctcttccgt tacctctgaa ttcccc 46
<210> 22
   <211> 45
   <212> DNA
   <213> Artificial
<220>
   <221> 5' Oligonukleotidprimer für die Herstellung von tTF-GCNGRCG
<400> 22
   catgccatgg gatcaggcac tacaaatact gtggcagcat ataat 45
<210> 23
   <211> 46
   <212> DNA
   <213> Artificial
<220>
   <221> 3' Oligonukleotidprimer für die Herstellung von tTF-GCNGRCG
<400> 23
   cgggatccta ttaaccacat ctaccgttgc agcctctgaa ttcccc 46
<210> 24
   <211> 45
   <212> DNA
   <213> Artificial
<220>
   <221> 5' Oligonukleotidprimer für die Herstellung von tTF-GCNGRCVSGCAGRC
<400> 24
   catgccatgg gatcaggcac tacaaatact gtggcagcat ataat 45
<210> 25
   <211> 67
   <212> DNA
   <213> Artificial
<220>
   <221> 3' Oligonukleotidprimer für die Herstellung von tTF-GCNGRCVSGCAGRC
<400> 25
   cgggatccta ttaacatcgt cctgcacatc cagaaacaca tcttccatta caacctctga 60
   attcccc 67
<210> 26
   <211> 45
   <212> DNA
   <213> Artificial
<220>
   <221> 5' Oligonukleotidprimer für die Herstellung von tTF-GCVLNGRMEC
<400> 26
   catgccatgg gatcaggcac tacaaatact gtggcagcat ataat 45
<210> 27
   <211> 55
   <212> DNA
   <213> Artificial
<220>
   <221> 3' Oligonukleotidprimer für die Herstellung von tTF-GCVLNGRMEC
<400> 27
   cgggatccta ttagcattcc atcctaccat ttaagacgca tcctctgaat tcccc 55
<210> 28
   <211> 45
   <212> DNA
   <213> Artificial
<220>
   <221> 5' Oligonukleotidprimer für die Herstellung von tTF-GALNGRSHAG
<400> 28
   catgccatgg gatcaggcac tacaaatact gtggcagcat ataat 45
<210> 29
   <211> 55
   <212> DNA
   <213> Artificial
<220>
   <221> 3' Oligonukleotidprimer für die Herstellung von tTF-GALNGRSHAG
<400> 29
   cgggatccta ttaaccagcg tgagatcttc catttaaagc acctctgaat tcccc 55
<210> 30
   <211> 45
   <212> PRT
   <213> Artificial
<220>
   <221> Aminosäuresequenz des Affinitäts-tags
<400> 30
   His His His His His His Ser Ser Gly Leu Val Pro Arg Gly Ser Gly
   1 5 10 15
   Met Lys Glu Thr Ala Ala Ala Lys Phe Glu Arg Gln His Met Asp Ser
   20 25 30
   Pro Asp Leu Gly Thr Asp Asp Asp Asp Lys Ala Met Gly
   35 40 45
<210> 31
   <211> 269
   <212> PRT
   <213> Artificial
<220>
   <221> Aminosäuresequenz von tTF-GRGDSP mit N-terminalem Affinitäts-tag
<400> 31
   His His His His His His Ser Ser Gly Leu Val Pro Arg Gly Ser Gly
   1 5 10 15
   Met Lys Glu Thr Ala Ala Ala Lys Phe Glu Arg Gln His Met Asp Ser
   20 25 30
   Pro Asp Leu Gly Thr Asp Asp Asp Asp Lys Ala Met Gly Ser Gly Thr
   35 40 45
   Thr Asn Thr Val Ala Ala Tyr Asn Leu Thr Trp Lys Ser Thr Asn Phe
   50 55 60
   Lys Thr Ile Leu Glu Trp Glu Pro Lys Pro Val Asn Gln Val Tyr Thr
   65 70 75 80
   Val Gln Ile Ser Thr Lys Ser Gly Asp Trp Lys Ser Lys Cys Phe Tyr
   85 90 95
   Thr Thr Asp Thr Glu Cys Asp Leu Thr Asp Glu Ile Val Lys Asp Val
   100 105 110
   Lys Gln Thr Tyr Leu Ala Arg Val Phe Ser Tyr Pro Ala Gly Asn Val
   115 120 125
   Glu Ser Thr Gly Ser Ala Gly Glu Pro Leu Tyr Glu Asn Ser Pro Glu
   130 135 140
   Phe Thr Pro Tyr Leu Glu Thr Asn Leu Gly Gln Pro Thr Ile Gln Ser
   145 150 155 160
   Phe Glu Gln Val Gly Thr Lys Val Asn Val Thr Val Glu Asp Glu Arg
   165 170 175
   Thr Leu Val Arg Arg Asn Asn Thr Phe Leu Ser Leu Arg Asp Val Phe
   180 185 190
   Gly Lys Asp Leu Ile Tyr Thr Leu Tyr Tyr Trp Lys Ser Ser Ser Ser
   195 200 205
   Gly Lys Lys Thr Ala Lys Thr Asn Thr Asn Glu Phe Leu Ile Asp Val
   210 215 220
   Asp Lys Gly Glu Asn Tyr Cys Phe Ser Val Gln Ala Val Ile Pro Ser
   225 230 235 240
   Arg Thr Val Asn Arg Lys Ser Thr Asp Ser Pro Val Glu Cys Met Gly
   245 250 255
   Gln Glu Lys Gly Glu Phe Arg Gly Arg Gly Asp Ser Asp
   260 265
<210> 32
   <211> 270
   <212> PRT
   <213> Artificial
<220>
   <221> Aminosäuresequenz von tTF-GNGRAHA mit N-terminalem Affinitäts-tag
<400> 32
   His His His His His His Ser Ser Gly Leu Val Pro Arg Gly Ser Gly
   1               5                   10                  15
   Met Lys Glu Thr Ala Ala Ala Lys Phe Glu Arg Gln His Met Asp Ser
               20                  25                  30
   Pro Asp Leu Gly Thr Asp Asp Asp Asp Lys Ala Met Gly Ser Gly Thr
           35                  40                  45
   Thr Asn Thr Val Ala Ala Tyr Asn Leu Thr Trp Lys Ser Thr Asn Phe
       50                  55                  60
   Lys Thr Ile Leu Glu Trp Glu Pro Lys Pro Val Asn Gln Val Tyr Thr
   65                  70                  75                  80
   Val Gln Ile Ser Thr Lys Ser Gly Asp Trp Lys Ser Lys Cys Phe Tyr
                   85                  90                  95
   Thr Thr Asp Thr Glu Cys Asp Leu Thr Asp Glu Ile Val Lys Asp Val
               100                 105                 110
   Lys Gln Thr Tyr Leu Ala Arg Val Phe Ser Tyr Pro Ala Gly Asn Val
           115                 120                 125
   Glu Ser Thr Gly Ser Ala Gly Glu Pro Leu Tyr Glu Asn Ser Pro Glu
       130                 135                 140
   Phe Thr Pro Tyr Leu Glu Thr Asn Leu Gly Gln Pro Thr Ile Gln Ser
   145                 150                 155                 160
   Phe Glu Gln Val Gly Thr Lys Val Asn Val Thr Val Glu Asp Glu Arg
                   165                 170                 175
   Thr Leu Val Arg Arg Asn Asn Thr Phe Leu Ser Leu Arg Asp Val Phe
               180                 185                 190
   Gly Lys Asp Leu Ile Tyr Thr Leu Tyr Tyr Trp Lys Ser Ser Ser Ser
           195                 200                 205
   Gly Lys Lys Thr Ala Lys Thr Asn Thr Asn Glu Phe Leu Ile Asp Val
       210                 215                 220
   Asp Lys Gly Glu Asn Tyr Cys Phe Ser Val Gln Ala Val Ile Pro Ser
   225                 230                 235                 240
   Arg Thr Val Asn Arg Lys Ser Thr Asp Ser Pro Val Glu Cys Met Gly
                   245                 250                 255
   Gln Glu Lys Gly Glu Phe Arg Gly Asn Gly Arg Ala His Ala
               260                 265                 270

## Beschreibung

Die vorliegende Erfindung betrifft Fusionspolypeptide, die aus mindestens zwei Peptiden zusammengesetzt sind. Ein Peptid besteht aus 3 bis 30 Aminosäuren und ermöglicht eine selektive Bindung des Fusionspolypeptides an Endothelzellen in Tumorgefäßen. Das andere Peptid besteht aus dem Gewebefaktor TF (Tissue Factor) oder einem Fragment davon, wobei der Gewebefaktor und das Fragment dadurch gekennzeichnet sind, daß sie die Blutgerinnung bei Bindung des Fusionspolypeptides an Endothelzellen in Tumorgefäßen aktivieren können. Die Peptide können entweder unmittelbar oder über einen Linker mit bis zu 15 Aminosäuren miteinander verbunden sein. Die Erfindung betrifft ferner die Verwendung dieser Fusionsproteine bei der antivaskulären Therapie von Tumorerkrankungen sowie deren Verwendung bei der Herstellung eines Arzneimittels zur Behandlung von Tumorerkrankungen.

### Hintergrund der Erfindung

Eine adäquate Neovaskularisierung ist eine Voraussetzung für ein progressives Tumorwachstum (1). Die Neoangiogenese ist insbesondere für die Aufrechterhaltung eines expansiven Tumorwachstums erforderlich, da nur so eine ausreichende Oxygenierung, die Versorgung des Tumors mit Nährstoffen und der Abtransport von Tumorabbauprodukten gewährleistet wird.

Zur Bekämpfung von Tumoren sind daher im Stand der Technik neben den anti-angiogenen Therapiestrategien, welche in den komplexen Prozeß des Wachstums und der Differenzierung der Blutgefäße eingreifen, auch antivaskuläre Therapiestrategien entwickelt worden, die auf eine Zerstörung der Tumorblutgefäße und einen damit verbundenen Tumor-Infarkt zielen.

Voraussetzung für diese Strategien ist die Identifizierung von Zielstrukturen im Gefäßendothel des Tumors, welche auf ruhenden Endothelzellen im normalen Gewebe nicht vorkommen. Solche spezifischen Zielstrukturen könnten benutzt werden, um Zytostatika oder bestimmte Toxine an die Gefäßendothelzellen des Tumors und weniger an die Tumorzellen selbst heranzubringen. Zielstrukturen, die für diesen Zweck verwendet werden könnten sind bFGF (basic fibroblast growth factor), VEGF (vascular endothelial growth factor) und VEGF Rezeptor 2 (VEGFR-2), Endoglin, Endosialin, eine Fibronektin-Isoform (ED-B Domäne), die Integrine αᵥβ₃, αᵥβ₅, α₁β₁ und α₁β₂, die Aminopeptidase N, das NG2 Proteoglykan und die Matrix Metalloproteinasen 2 und 9 (MMP 2 und 9) (2-13). Arap et al. (8) koppelten beispielsweise Peptide, die alpha1-Integrine spezifisch binden, an einen Wirkstoff, der im Stand der Technik für die Chemotherapie verwendet worden war (Doxorubicin). Im Tiermodell zeigte sich, daß die antineoplastische Wirkung des Doxorubicins durch Kopplung an die Peptide verbessert werden konnte.

Ein alternativer antivaskulärer Therapieansatz besteht in der selektiven Aktivierung der Blutgerinnung in Tumorgefäßen, um eine Tumornekrose zu induzieren. Beispielsweise wurde ein bispezifisches F(ab')2-Antikörper Fragment erzeugt, das gegen löslichen Gewebefaktor (truncated tissue factor, tTF) und ein MHC-Klasse II Antigen gerichtet ist. Nach experimenteller Induktion des Antigens in Tumorendothelzellen, konnte eine antivaskuläre Therapie durch Verabreichung des Antikörpers in einem murinen Neuroblastom Modell gezeigt werden (14 und 47). In einer zweiten Studie der gleichen Arbeitsgruppe wurde ein Immunkonjugat eingesetzt, welches tTF gezielt an einen natürlich vorkommenden Marker des Tumorgefäß-Endothels, VCAM-1 (vascular cell adhesion molecule-1), koppelt (15).

In einem sehr ähnlichen Ansatz wurde ein Antikörperfragment (scFv), welches spezifisch für die onkofetale ED-B Domäne ist, mit tTF fusioniert. Die generierten Fusionsproteine, scFv-tTF, führten zu einem vollständigen und selektiven Infarkt in verschiedenen Tumoren im Mausmodell (16).

Alternativ dazu wurde tTF an einen Inhibitor des Prostata-spezifischen Membran Antigens gekoppelt (17). Dieses Fusionsprotein induzierte eine selektive Infarktnekrose in einem Ratten Prostata Modell nach intravenöser Gabe. Die Gabe dieses Fusionsproteins führte in Kombination mit einer niedrig-dosierten zytotoxischen Substanz (Doxorubicin) zu einer massiven Tumorregression bis hin zur kompletten Tumor-Eradikation (17). Andere tTF-Fusionsproteine, bestehend aus Antikörperfragmenten gegen VEGFR, Endoglin und VCAM-1, wurden kürzlich beschrieben (18).

Die im Stand der Technik für die antivaskuläre Tumortherapie hergestellten Moleküle weisen jedoch Nachteile auf. Es ist insbesondere davon auszugehen, daß diese Moleküle aufgrund ihrer Größe immunogen sind. Die Behandlung von Säugetieren mit diesen Molekülen wird daher eine Immunreaktion gegen die Moleküle auslösen, wodurch eine wiederholte Verabreichung der Moleküle unmöglich wird.

Die Größe des Kopplungspartners, mittels dessen der Peptidanteil, der die Blutgerinnung aktivieren kann, auf das Tumorgewebe gerichtet werden soll, kann ferner die für die Blutgerinnung wesentliche Bildung des makromolekularen Enzym- und Substratkomplexes Faktor VIIa/FX sterisch hindern. Die Bildung des Komplexes kann auch dadurch behindert werden, daß das Peptid, welches die Blutgerinnung aktivieren kann, durch die relativ großen Fusionspartner eine geänderte Konformation aufweist.

Im Stand der Technik (WO 03/035688) sind weiterhin Fusionspolypeptide bekannt, in denen eine selektive Bindungsdomäne, z.B. eine an Integrine bindende Domäne aus Fibronektin, die z.B. RGD-Peptide umfasst, oder das Dipeptid D-β-E, das an PSMA (Prostata-spezifisches Membran-Antigen) bindet, an den N-Terminus eines Tissue-Faktor-Polypeptids gekoppelt ist. Obwohl *in vitro* eine amidolytische und proteolytische Wirkung gezeigt werden konnte, zeigten die Konstrukte *in vivo*, selbst in Kombination mit Faktor VIIa nur äußerst schwache Anti-Tumorwirkung. Erst in Kombination mit Doxycyclin überlebten die Tier länger.

Hu et al. (46) beschreiben verschiedene Fusionsproteine und deren Verwendung zur Erzeugung von Thrombosen in Tumorgefäßen, u.a. ein Fusionsprotein aus einem Oligopeptid mit 9 Aminosäuren, das die Sequenz RGD enthält, das an die verkürzte Form des Tissue-Faktors gekoppelt wurde. Auch hier wurden die RGD-Peptide mit dem N-Terminus von tTF zu RGD-tTF verknüpft. Funktionsanalysen ergaben, dass das RGD enthaltende Fusionsprotein keine signifikante Hemmung des Tumorwachstums verursachte.

Die im Stand der Technik bekannten Konstrukte wurden daher so aufgebaut, dass die selektive Bindungsdomäne mit dem N-Terminus des Tissue-Faktor-Polypeptids verknüpft wurde. Es wurde sogar betont, dass dieser Aufbau gewählt werden muß, da der N-Terminus aufgrund von Strukturmodellen ein besonders günstiger Ort für eine Verknüpfung sei, welche die Initiation der Thrombose nicht hemme.

### Zusammenfassung der Erfindung

Gegenüber dem Stand der Technik stellt sich daher die Aufgabe, alternative thrombogene Wirkstoffe zur Verfügung zu stellen, die das Tumorwachstum in vivo effektiv hemmen können.

Dieses Problem wurde nunmehr durch Fusionspolypeptide gelöst, welche ein Peptid von 3 - 30 Aminosäuren, daß eine selektive Bindung des Fusionspolypeptides an Tumorgefäßendothelzellen ermöglicht, und den Gewebefaktor TF (Tissue Factor) oder ein Fragment davon umfassen, wobei der Gewebefaktor und das Fragment dadurch gekennzeichnet sind, daß sie die Blutgerinnung bei Bindung des Fusionspolypeptides an Tumorgefäßendothelzellen aktivieren können, wobei diese Peptide entweder unmittelbar oder über einen Linker mit bis zu 15 Aminosäuren aneinander gekoppelt sind. Dabei ist das Peptid, das eine selektive Bindung des Fusionspolypeptides an Tumorgefäßendothelzellen ermöglicht, an den C-Terminus des Peptids, das die Blutgerinnung bei Bindung des Fusionspolypeptides an Tumorgefäßendothelzellen aktivieren kann, gekoppelt. Die vorliegende Erfindung betrifft ferner Arzneimittel, welche entsprechende Fusionspolypeptide enthalten und deren Verwendung zur Behandlung von Tumoren.

### Beschreibung der Figuren

- Fig.1:: Schematische Darstellung der Bindung der Fusionsproteine tTF-RGD und tTF-NGR an αᵥβ₃ und CD13. Durch die Spezifität der RGD-Sequenz für das αᵥβ₃-Integrin und der NGR-Sequenz für CD13 (Aminopeptidase N) wird die Tumorselektivität erreicht. Diese Rezeptoren werden selektiv und spezifisch in hoher Dichte auf Tumor-Endothelzellen exprimiert, nicht aber auf ruhenden Endothelzellen im normalen Gewebe (abgesehen von wenigen Ausnahmen). Die Darstellung der Fusionsproteine ist stark schematisiert und erlaubt keine Aussage hinsichtlich der Primärsequenz.

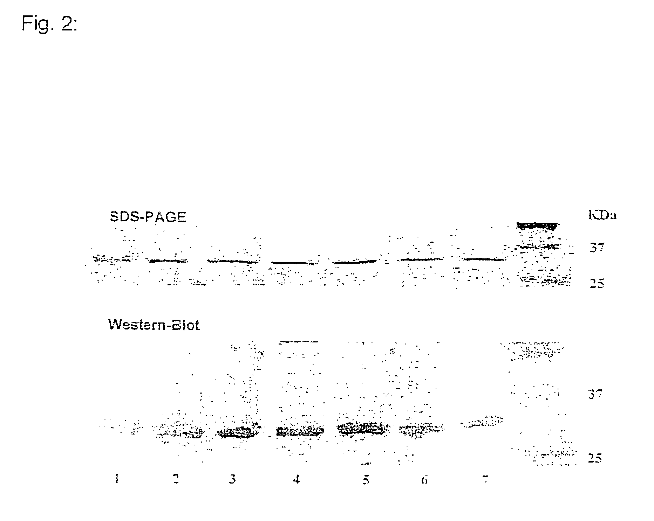

- Fig.2:: SDS-PAGE und Western-Blot-Analyse von rekombinantem tTF₁₋₂₁₈ und tTF-Fusionsproteinen. Die Reinheit des tTF und der tTF-Fusionsproteine wurde nach Extraktion aus *E.coli* (BL21 DE3) und "refolding" über einen linearen Harnstoff-Gradienten (6M - 1M) mittels SDS-PAGE mit anschließender Coomassie-blue-Färbung kontrolliert. Die Identität der Proteine wurde mittels Western-Blot unter Verwendung eines monoklonalen anti-Tissue-Factor-Antikörpers (Klon VIC7, American Diagnostics) verifiziert. Belegung der einzelnen Bahnen: 1=tTF; 2=tTF-RGD; 3=tTF-NGR; 4=tTF-cycloNGR1; 5=tTF-cycloNGR2; 6=tTF-cycloNGR3; 7=tTF-GALNGRSHAG; M = Molekulargewichtsmarker.
- Fig.3:: Bestimmung der Michaeliskonstanten (Km) für die Aktivierung von FX durch FVIIa/tTF₁₋₂₁₈ bzw. FVIIa/tTF₁₋₂₁₈-Fusionsproteine. Die Parameter der Michaelis-Menten-Kinetik wurden nach der von Ruf angegebenen Methode berechnet (45).
- Fig.4:: Bindung von tTF, tTF-RGD und tTF-NGR an das Integrin αᵥβ₃. Die Bindung von 0,1µM tTF, tTF-RGD und tTF-NGR an immobilisiertes αᵥβ₃ wurde mit einem polyklonalen Antikörper gegen humanen TF (American Diagnostica) in einem ELISA quantifiziert. Die Ergebnisse sind als Median und Interquartilbereich dargestellt. Die Unterschiede der Bindung zwischen tTF-RGD und tTF bzw. zwischen tTF-NGR und tTF waren statistisch signifikant (p<0,001, Mann-Whitney-Test).
- Fig.5:: Spezifität der Bindung von tTF-RGD an das Integrin αvβ3. Die Bindung von tTF-RGD (0,1µM) an immobilisiertes αᵥβ₃ wurde mittels kompetitiver Inhibition mit dem synthetischen Peptid GRGDSP (1-10µM) signifikant gehemmt (p<0,001, Mann-Whitney-Test für beide RGD Peptidkonzentrationen).
- Fig.6:: Bindung von tTF und tTF-RGD an humane Endothelzellen. A: FACS Analyse von Endothelzellen, die mit 0,1 µM tTF (2) oder mit 0,1 µM tTF-RGD (3) für 60 min. bei 4°C inkubiert wurden. B: Durch kompetitive Inhibition des tTF-RGD Fusionsproteins mit 1 µM GRGDSP war eine 75%ige Reduktion der Bindung darstellbar (4). Die Kurven 1 in A und B zeigen die negative Kontrolle.
- Fig.7:: Inhibition eines als Xenotransplantat in thymuslosen Nackt-Mäusen wachsenden menschlichen Lungenkarzinoms (CCL185) durch intravenöse Therapie mit tTF-Fusionsproteinen (tTF-RGD, n=6; tTF-NGR, n=6) im Vergleich zum Wachstum der Tumoren bei Infusion von physiologischer Kochsalzlösung (NaCl, n=8) oder tTF (n=1). Die senkrechten Pfeile kennzeichnen die Zeitpunkte der Injektionen mit den jeweiligen Substanzen.
- Fig.8:: Inhibition und Teilremission eines als Xenotransplantat in thymuslosen Nackt-Mäusen wachsenden menschlichen malignen Melanoms (M21) durch intravenöse Therapie mit tTF-Fusionsproteinen (tTF-RGD, n=3; tTF-NGR, n=3) im Vergleich zum Wachstum der Tumoren bei Infusion von physiologischer Kochsalzlösung (NaCl, n=4) oder tTF (n=4). Die senkrechten Pfeile kennzeichnen die Zeitpunkte der Injektionen mit den jeweiligen Substanzen.

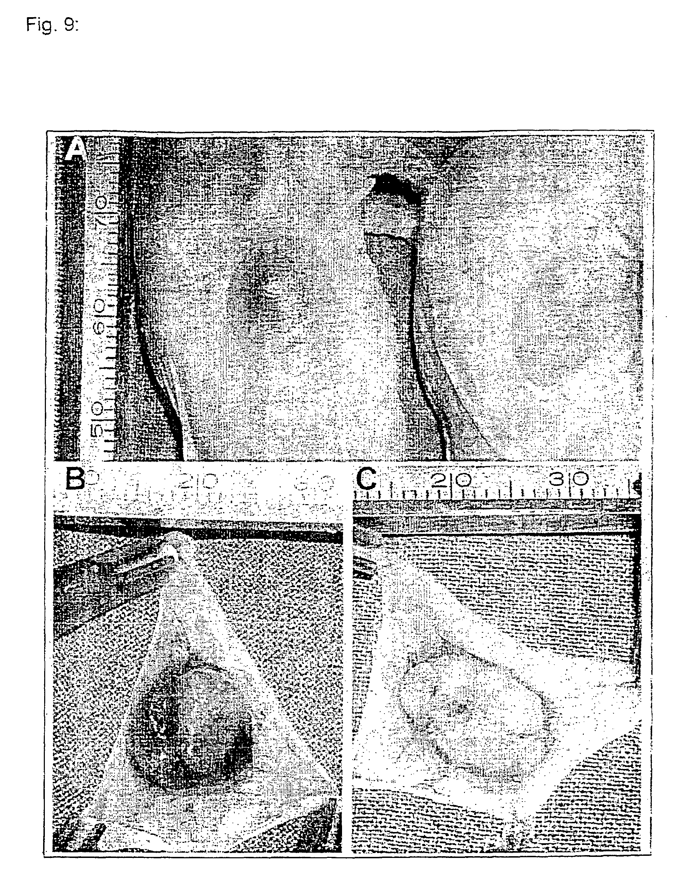

- Fig.9:: Makroskopische in vivo-Aufnahme einer tumortragenden Maus 20 min. nach Injektion des tTF-NGR Fusionsproteins (A, linke Bildhälfte) bzw. NaCl (A, rechte Bildhälfte). Das makroskopische Bild mit bläulich-livider Verfärbung des Tumors nach Injektion von tTF-NGR deutet auf eine Tumornekrose hin. Nach 60 min. wurden beide Mäuse exsanguiniert, der Tumor *in toto* exstipiert und histologisch untersucht. In B ist die hämorrhagische Imbibierung des mit tTF-NGR behandelten Tumors als Zeichen der sekundären Einblutung infolge der beginnenden Tumornekrose sichtbar. Im Gegensatz hierzu scheint der mit NaCl behandelte Tumor vital zu sein (C).

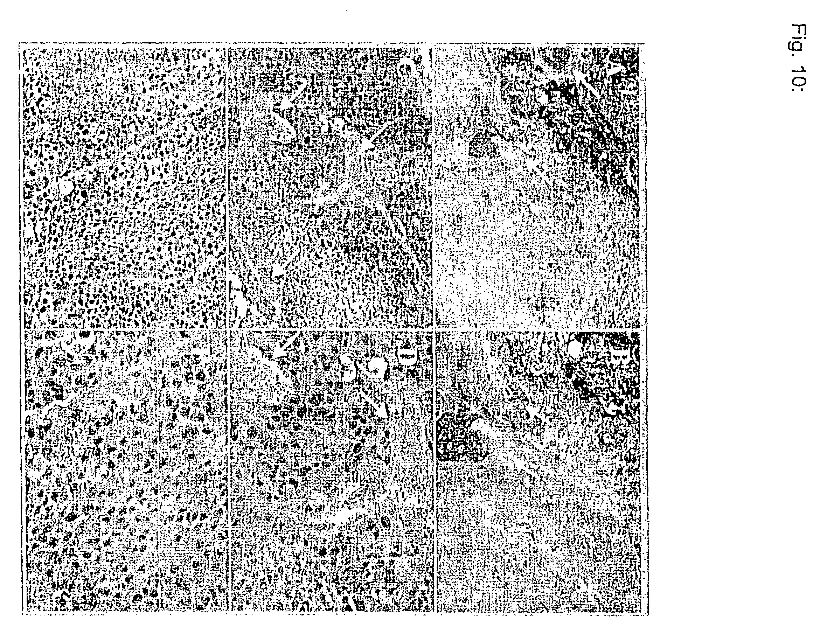

- Fig.10:: Histologie des Melanom-Tumors 1 Stunde nach intravenöser Injektion von tTF-RGD (A und B), tTF-NGR (C und D) und Kochsalz (E und F) in die Schwanzvene der tumortragenden Nacktmaus. Die Blutgefässe erscheinen bei den mit den tTF Fusionsproteinen behandelten Tumoren thrombotisch verschlossen (Pfeile). Im Versorgungsgebiet des durch ein Blutgerinnsel verschlossenen Gefäßes können ausgedehnte Tumor-Nekrosen beobachtet werden (A-D). Photographiert wurden repräsentative Areale der Tumoren (A, C und E: 200fache Vergrößerung, B, D und F 400fache Vergrößerung; HE-Färbung (Färbung z.B. beschrieben in H.C. Burck, Histologische Technik - Leitfaden für die Herstellung mikroskopischer Präparate in Unterricht und Praxis 5. Auflage, Thieme Verlag, Stuttgart 1982, Seiten 109 ff.).
- Fig.11:: Repräsentative Histologien von Herz (A), Niere (B), Leber (C) und Lunge (D) 1 Stunde nach Injektion von 4mg/kg KG tTF-NGR. In keinem dieser Organe waren mikroskopisch Thrombosen oder Nekrosen nachweisbar. (HE-Färbung; 200fache Vergrößerung).
- Fig.12:: Aminosäuresequenz des humanen Gewebefaktors (TF).
- Fig.13:: Aminosäuresequenz des trunkierten humanen Gewebefaktors tTF₁₋₂₁₈ (im Rahmen der vorliegenden Anmeldung auch kurz als tTF bezeichnet).
- Fig.14:: Aminosäuresequenz des Fusionspolypeptids tTF-GRGDSP (auch kurz als tTF-RGD bezeichnet).
- Fig.15:: Aminosäuresequenz des Fusionspolypeptids tTF-GNGRAHA (auch kurz als tTF-NGR bezeichnet).
- Fig.16:: Aminosäuresequenz des Fusionspolypeptids tTF-GALNGRSHAG. Fig.17: Aminosäuresequenz des Fusionspolypeptids tTF-GCNGRCG (auch kurz als tTF-cycloNGR1 bezeichnet).
- Fig.18:: Aminosäuresequenz des Fusionspolypeptids tTF-GCNGRCVSGCAGRC (auch kurz als tTF-cycloNGR2 bezeichnet).
- Fig.19:: Aminosäuresequenz des Fusionspolypeptids tTF-GCVLNGRMEC (auch kurz als tTF-cycloNGR3 bezeichnet).
- Fig.20:: Nukleotidsequenz des trunkierten humanen Gewebefaktors tTF₁₋₂₁₈ (im Rahmen der vorliegenden Anmeldung kurz als tTF bezeichnet).
- Fig.21:: Nukleotidsequenz des Fusionspolypeptids tTF-GRGDSP (auch kurz als tTF-RGD bezeichnet).
- Fig.22:: Nukleotidsequenz des Fusionspolypeptids tTF-GNGRAHA (auch kurz als tTF-NGR bezeichnet).
- Fig.23:: Nukleotidsequenz des Fusionspolypeptids tTF-GALNGRSHAG.
- Fig.24:: Nukleotidsequenz des Fusionspolypeptids tTF-GCNGRCG (auch kurz als tTF-cycloNGR1 bezeichnet).
- Fig.25:: Nukleotidsequenz des Fusionspolypeptids tTF-GCNGRCVSGCAGRC (auch kurz als tTF-cycloNGR2 bezeichnet).
- Fig.26:: Nukleotidsequenz des Fusionspolypeptids tTF-GCVLNGRMEC (auch kurz als tTF-cycloNGR3 bezeichnet).
- Fig.27:: Nukleotidsequenz der Oligonukleotide zur Herstellung von tTF₁₋₂₁₈. A: 5'-Primer; B: 3'-Primer.
- Fig.28:: Nukleotidsequenz der Oligonukleotide zur Herstellung von tTF-GRGDSP. A: 5'-Primer; B: 3'-Primer.
- Fig.29:: Nukleotidsequenz der Oligonukleotide zur Herstellung von tTF-GNGRAHA. A: 5'-Primer; B: 3'-Primer.
- Fig.30:: Nukleotidsequenz der Oligonukleotide zur Herstellung von tTF-GCNGRCG. A: 5'-Primer; B: 3'-Primer.
- Fig.31:: Nukleotidsequenz der Oligonukleotide zur Herstellung von tTF-GCNGRCVSGCAGRC. A: 5'-Primer; B: 3'-Primer.
- Fig.32:: Nukleotidsequenz der Oligonukleotide zur Herstellung von tTF-GCVLNGRMEC. A: 5'-Primer; B: 3'-Primer.
- Fig.33:: Nukleotidsequenz der Oligonukleotide zur Herstellung von tTF-GALNGRSHAG. A: 5'-Primer; B: 3'-Primer.

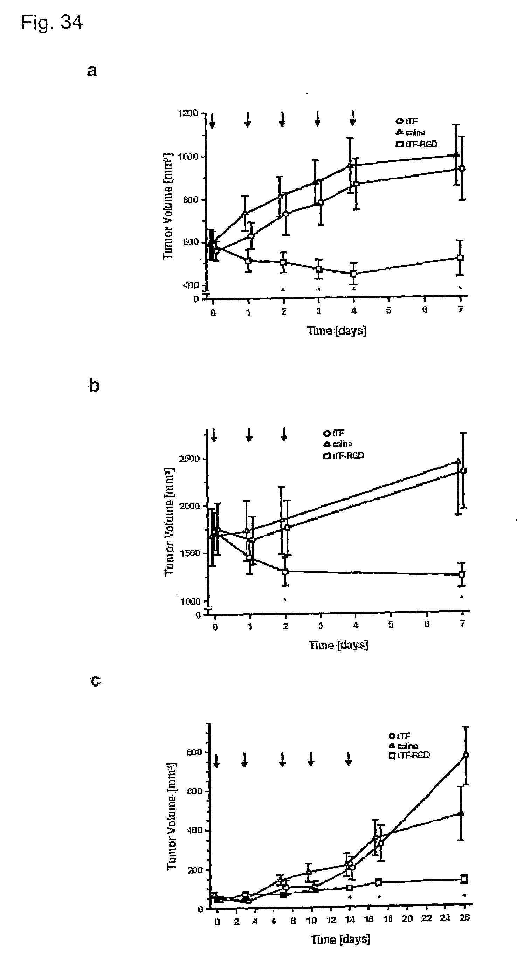

- Fig.34:: a: Inhibition und Teilremission eines als Xenotransplantat in thymuslosen Nackt-Mäusen wachsenden menschlichen malignen Melanoms (M21) durch intravenöse Therapie mit tTF-Fusionsproteinen (tTF-RGD, n=7) im Vergleich zum Wachstum der Tumoren bei Infusion von physiologischer Kochsalzlösung (NaCl, n=9) oder tTF (n=11). Die senkrechten Pfeile kennzeichnen die Zeitpunkte der Injektionen mit den jeweiligen Substanzen. b: Inhibition eines als Xenotransplantat in thymuslosen Nackt-Mäusen wachsenden menschlichen Fibrosarkoms (HT1080) durch intravenöse Therapie mit tTF-Fusionsproteinen (tTF-RGD, n=12) im Vergleich zum Wachstum der Tumoren bei Infusion von physiologischer Kochsalzlösung (NaCl, n=15) oder tTF (n=14). Die senkrechten Pfeile kennzeichnen die Zeitpunkte der Injektionen mit den jeweiligen Substanzen. c: Inhibition eines als Xenotransplantat in thymuslosen Nackt-Mäusen wachsenden menschlichen Lungenkarzinoms (CCL185) durch intravenöse Therapie mit tTF-Fusionsproteinen (tTF-RG3D, n=11) im Vergleich zum Wachstum der Tumoren bei Infusion von physiologischer Kochsalzlösung (NaCl, n=10) oder tTF (n=5). Die senkrechten Pfeile kennzeichnen die Zeitpunkte der Injektionen mit den jeweiligen Substanzen. Die statistische Signifikanz wurde jeweils mit dem Mann-Whitney-Test für unabhängige Gruppen untersucht, wobei P-Werte unter 0,05 als signifikant betrachtet wurden. * gibt die statistische Signifikanz des Unterschieds zwischen tTF-RGD und Puffer an.

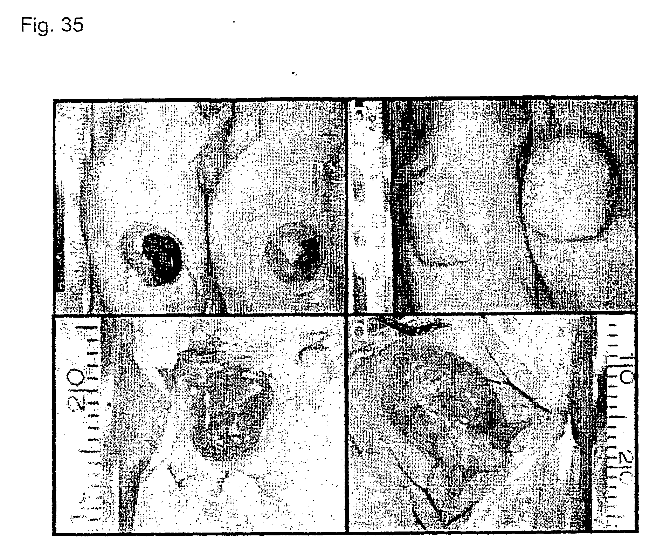

- Fig.35:: Makroskopische Aufnahme einer einen M21-Tumor tragenden Maus nach Ende der Behandlung (Tag 7) mit tTF-RGD Fusionsprotein (A, C) bzw. NaCl (B, D). Der Größenunterschied und das unterschiedliche Erscheinungsbild der tTF-RGD-behandelten Tumoren, die im Gegensatz zu dem vital erscheinenden Kontrolltumor klare Zeichen von Nekrose zeigen, ist deutlich erkennbar.

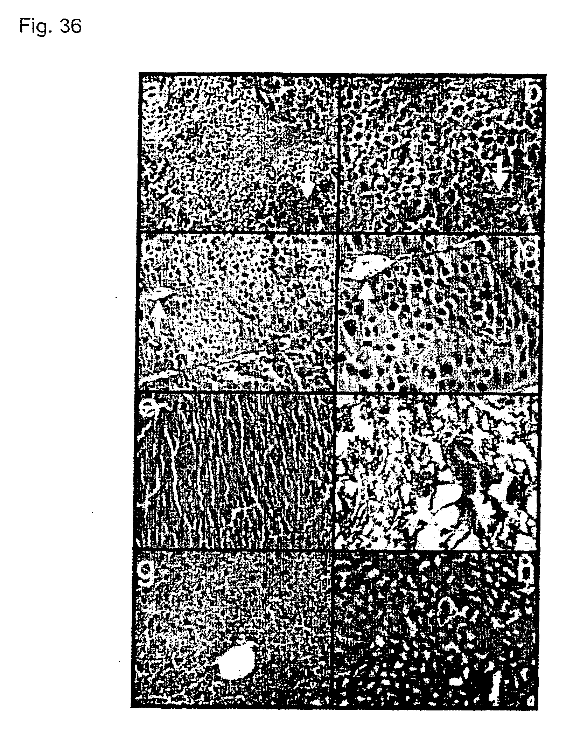

- Fig.36:: H-E-Färbung von Tumoren und Organen von mit tTF-RGD und physiologischer Kochsalzlösung behandelten Mäusen In mit tTF-RGD behandelten Tieren wurde starke Thrombose und Nekrose von Tumorzellen beobachtet (A: 200x, B: 400x). Pfeile zeigen Beispiele der Thrombosen in Blutgefäßen des Tumors. In mit Kochsalzlösung behandelten Tieren tritt keine offentsichtliche Thrombose oder Nekrose auf (C:200x, D: 400x). Pfeile zeigen intakte Blöugefäße des Tumors mit einigen Erythrocyten. Herz (E), Lunge (F), Leber (G), und Niere der mit tTF-RGD behandelten Tiere zeigten keine sichtbare Thrombose oder Nekrose.

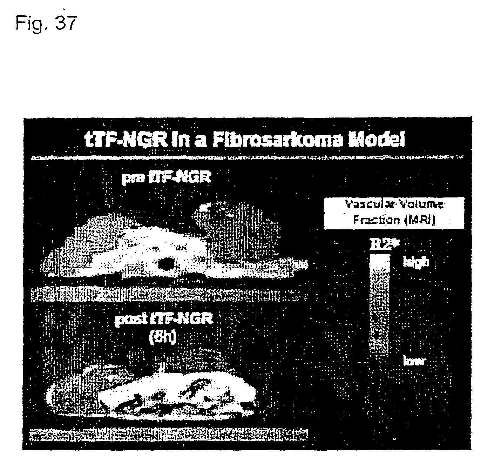

- Fig.37:: Wirkung von tTF-NGR in einem Fibrosarkom-Modell Fibrosarkom (HT1080) tragende Mäuse wurden ohne (pre tTF-NGR) bzw. 6 Stunden nach (post tTF-NGR) i.v. Gabe von tTF-NGR durch Kernspintomographie (MRI) untersucht. Gezeigt ist die hohe (high) oder niedrige (low) vaskuläre Volumenfraktion (Vascular volume fraction).

### Detaillierte Beschreibung der Erfindung

Diese Probleme aus dem Stand der Technik wurden nunmehr durch Fusionspolypeptide gelöst, die folgende Peptide umfassen:
a) ein Peptid von 3 bis 30 Aminosäuren, das eine selektive Bindung des Fusionspolypeptides an Tumorgefäßendothelzellen ermöglicht; und
b) den Gewebefaktor TF (Tissue Factor) oder ein Fragment davon, wobei der Gewebefaktor und das Fragment dadurch gekennzeichnet sind, daß sie die Blutgerinnung bei Bindung des Fusionspolypeptides an Tumorgefäßendothelzellen aktivieren können,
wobei die Peptide a) und b) entweder unmittelbar oder über einen Linker mit bis zu 15 Aminosäuren aneinander gekoppelt sind und das Peptid, das eine selektive Bindung des Fusionspolypeptides an Tumorgefäßendothelzellen ermöglicht, an den C-Terminus des Peptids, das die Blutgerinnung bei Bindung des Fusionspolypeptides an Tumorgefäßendothelzellen aktivieren kann, gekoppelt ist. Die vorliegende Erfindung betrifft ferner Arzneimittel, welche entsprechende Fusionspolypeptide enthalten und deren Verwendung zur Behandlung von Tumoren.

Die erfindungsgemäßen Fusionspolypeptide können neben den Sequenzen a) und b) weitere Sequenzen umfassen, soweit diese die sterische Konformation des Fusionspolypeptids nicht beeinträchtigen und die Bildung des die Blutgerinnung auslösenden Enzym- und Substratkomplexes nicht hindern. Die erfindungsgemäßen Fusionspolypeptide können beispielsweise die Sequenzen eines His-Tags enthalten, welche die rekombinante Expression und Reinigung des Peptids vereinfachen (vgl. Beispiele). Die Gegenwart dieser Sequenzen ist jedoch nicht notwendig. Gemäß einer bevorzugten Ausführungsform der Erfindung besteht das Fusionspolypeptid daher aus:
a) einem Peptid von 3 bis 30 Aminosäuren, das eine selektive Bindung des Fusionspolypeptides an Tumorgefäßendothelzellen ermöglicht; und
b) dem Gewebefaktor TF (Tissue Factor) oder einem Fragment davon, wobei der Gewebefaktor und das Fragment dadurch gekennzeichnet sind, daß sie die Blutgerinnung bei Bindung des Fusionspolypeptides an Tumorgefäßendothelzellen aktivieren können,
wobei die Peptide a) und b) entweder unmittelbar oder über einen Linker mit bis zu 15 Aminosäuren aneinander gekoppelt sind. Gemäß einer besonders bevorzugten Ausführungsform der Erfindung besteht das Fusionspolypeptid aus:
a) einem Peptid von 3 bis 30 Aminosäuren, das eine selektive Bindung des Fusionspolypeptides an Tumorgefäßendothelzellen ermöglicht; und
b) dem Gewebefaktor TF (Tissue Factor) oder einem Fragment davon, wobei der Gewebefaktor und das Fragment dadurch gekennzeichnet sind, daß sie die Blutgerinnung bei Bindung des Fusionspolypeptides an Tumorgefäßendothelzellen aktivieren können,
wobei die Peptide a) und b) aneinander gekoppelt sind.

Erfindungsgemäß konnte somit überraschenderweise gezeigt werden, daß Fusionspolypeptide aus einem besonders kleinen Peptid, das eine selektive Bindung des Fusionspolypeptides an Tumorgefäßendothelzellen ermöglicht und einem Peptid, daß die Blutgerinnung bei der Bindung des Fusionspolypeptides an Tumorgefäßendothelzellen aktivieren kann, für die antivaskuläre Tumortherapie besonders vorteilhaft sind. Die geringe Größe des Polypeptides, das die Bindung an die Tumorgefäßendothelzellen ermöglicht, verbessert die Ausrichtung des Fusionsproteins zur Phospholipidmembran der Endothelzelle. Die Bildung des für die Blutgerinnung wesentlichen Enzym-/Substratkomplexes wird nicht sterisch behindert und der Gewebefaktor TF, der die Blutgerinnung aktivieren kann, erfährt keine Konformationsveränderung.

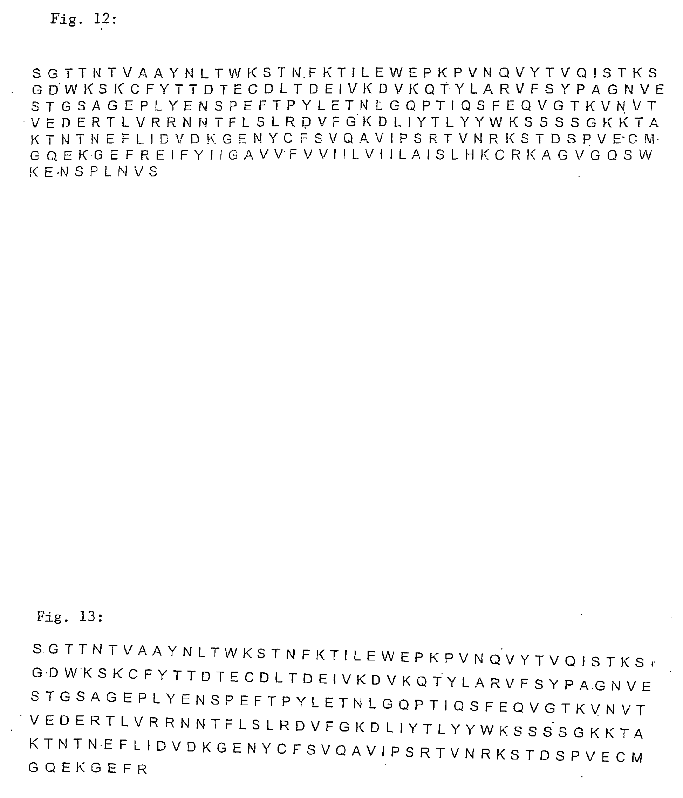

Gemäß einer bevorzugten Ausführungsform der vorliegenden Erfindung handelt es sich bei dem Peptid, das die Blutgerinnung bei Bindung des Fusionspolypeptides an Tumorgefäßendothelzellen aktivieren kann um den Gewebefaktor TF mit der in SEQ ID NO:1 (Fig. 12) dargestellten Aminosäure-Sequenz. Erfindungsgemäß sind ferner Gewebefaktor-Sequenzen umfaßt, die eine Aminosäure-Homologie von mindestens 70% oder mindestens 80% zu der SEQ ID NO:1 (Fig. 12) aufweisen, wobei Sequenzen mit einer Homologie von mindestens 95 % besonders bevorzugt sind. Die Bestimmung des Homologiegrades erfolgt, indem man die beiden Sequenzen übereinander schreibt, wobei vier Lücken auf einer Länge von 100 Aminosäuren möglich sind, um größtmögliche Übereinstimmung der zu vergleichenden Sequenzen zu erzielen (vgl. auch Dayhoff, Atlas of Protein Sequence and Structure, 5, 124, 1972). Anschließend wird der Prozentsatz der Aminosäurereste der kürzeren der beiden Aminosäureketten ermittelt, der identischen Aminosäureresten auf der anderen Kette gegenübersteht.

Das Peptid, das die Blutgerinnung in Tumorgefäßen bei Bindung des Fusionspolypeptides an Tumorgefäßendothelzellen aktivieren kann, kann ferner ein Fragment des Gewebefaktors TF oder ein Fragment einer zu TF homologen Sequenz sein. Vorzugsweise weist das Fragment die in SEQ ID NO:2 (Fig. 13) dargestellte Sequenz auf. Die in SEQ ID NO:2 (Fig. 13) dargestellte Sequenz (tTF₁₋₂₁₈ oder kurz tTF) umfaßt die N-terminalen 218 Aminosäuren von TF. Ferner können erfindungsgemäß auch Fragmente von tTF verwendet werden, denen gegenüber tTF mehrere Aminosäuren am N-Terminus oder C-Terminus fehlen. Beispielsweise können Fragmente verwendet werden, denen bis zu 10 Aminosäuren am N-Terminus fehlen (tTF₁₁₋₂₁₈). Des weiteren können Fragmente verwendet werden, denen bis zu 8 Aminosäuren am C-Terminus fehlen (tTF₁₋₂₁₀), wie z.B. (tTF₁₋₂₁₄).

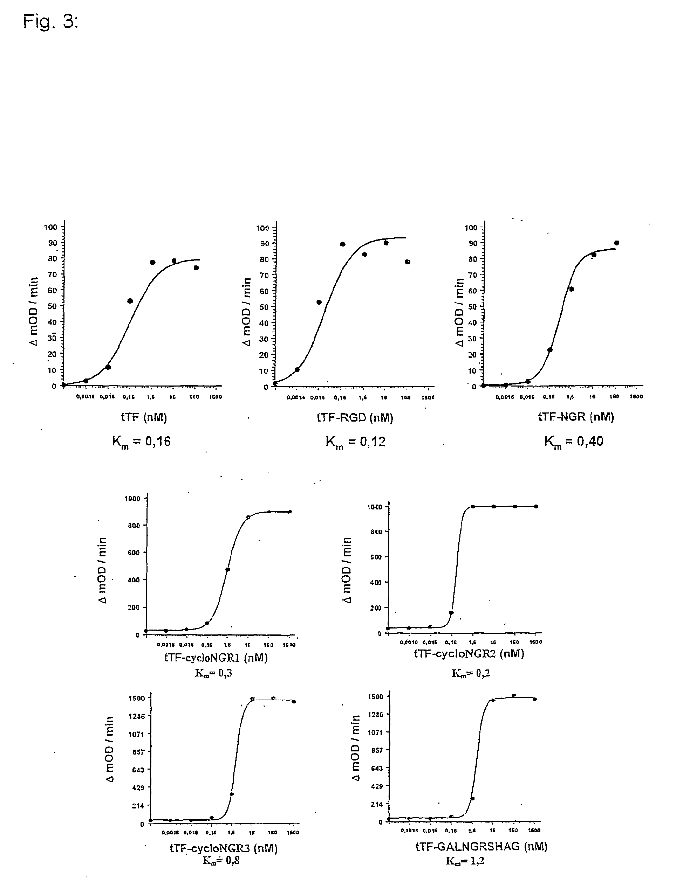

Die vorliegende Erfindung betrifft Fusionspolypeptide, in denen das Peptid, das eine selektive Bindung an die Endothelzellen der Tumorgefäße ermöglicht, an den C-Terminus des Peptids gekoppelt ist, das die Blutgerinnung aktivieren kann. Unter "Tumorgefäßendothelzellen" bzw. "Endothelzellen in Tumorgefäßen" werden dabei erfindungsgemäß Zellen verstanden, die die Blutgefäße in einem Tumor auskleiden. Erfindungsgemäß wurde festgestellt, daß diese Anordnung eine Ausrichtung des Fusionsproteins senkrecht zur Phospholipidmembran der Endothelzelle gewährleistet, welche für die Auslösung der Blutgerinnung besonders vorteilhaft ist. Diese Orientierung entspricht der natürlichen Ausrichtung des TF bei der Induktion der Blutgerinnung. Wie in Figur 3 gezeigt, ergeben sich für alle so hergestellten Konstrukte bezüglich der Aktivierung von Faktor X durch FVIIa/tTF₁₋₂₁₈ bzw. FVIIa/tTF₁₋₂₁₈-Fusionsproteine sehr ähnliche Michaelis-Menten-Kinetiken. Demgegenüber wurde im Stand der Technik das die Gerinnung aktivierende Peptid an den C-Terminus des Targeting-Moleküls gekoppelt (vgl. (16)). Die erfindungsgemäßen Fusionspolypeptide unterscheiden sich somit grundsätzlich von denen im Stand der Technik genannten Peptiden.

Das Peptid, das eine selektive Bindung des Fusionspolypeptides an Tumorgefäßendothelzellen ermöglicht, kann ein beliebiges Peptid sein, das eine Länge von 3 - 30 Aminosäuren aufweist und Tumorgefäßendothelzellen mit hoher Spezifität bindet. Entsprechende Peptide können aus Peptidbibliotheken durch im Stand der Technik übliche Verfahren isoliert werden. Sie können - je nach gewählter Peptidbibliothek - eine lineare oder cyclische Struktur aufweisen.

Gemäß einer Ausführungsform der vorliegenden Erfindung umfassen die Peptide, die eine selektive Bindung des Fusionspolypeptides an Tumorgefäßendothelzellen ermöglichen, die Aminosäuresequenz RGD oder NGR. Beide Sequenzen waren im Stand der Technik für Ihre spezifische Bindung an Integrine, insbesondere αᵥβ₃ und αᵥβ₅ Integrine (RGD-Peptide), sowie als Zelladhäsionsmotive (NGR-Peptide) bekannt (vgl. (8)). Erfindungsgemäß wurde nunmehr überraschenderweise gezeigt, daß diese Peptide insbesondere dazu geeignet sind, Teil eines Fusionspolypeptides zu sein, dessen anderer Teil ein Peptid ist, das die Blutgerinnung in Tumoren bei Bindung des Fusionspolypeptides an Tumorgefäßendothelzellen aktivieren kann.

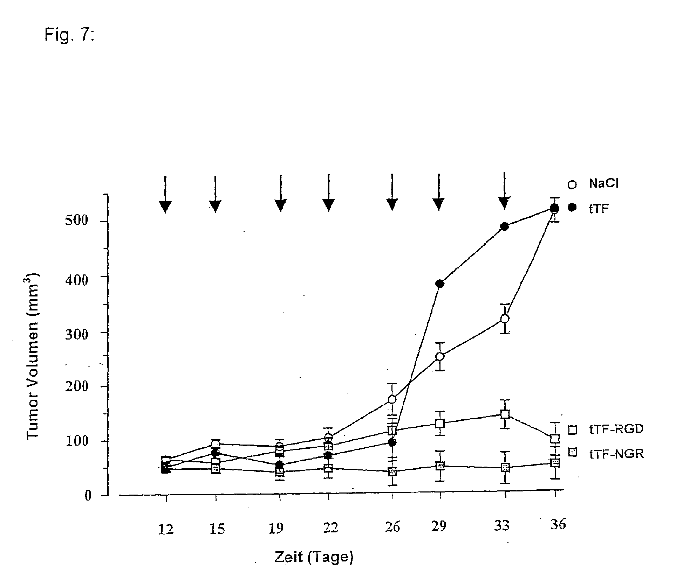

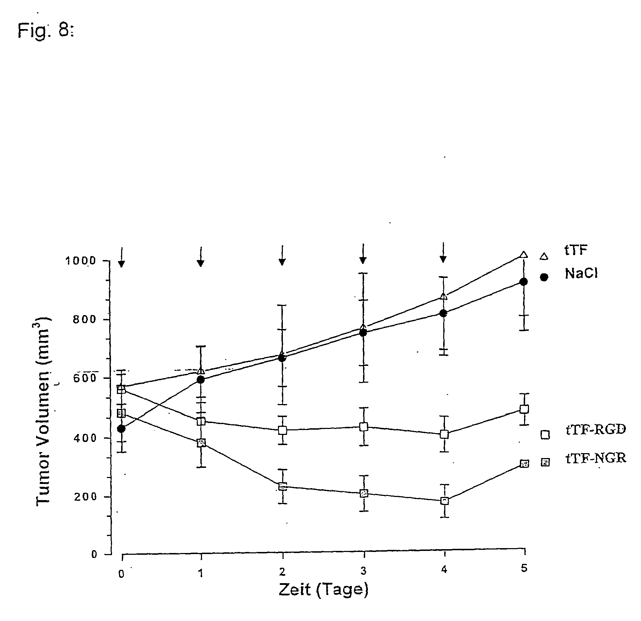

Besonders vorteilhafte Wirkungen wurden mit den linearen Peptiden mit den Sequenzen GRGDSP, GNGRAHA und GALNGRSHAG und den zyklischen Peptiden mit den Sequenzen GCNGRCG, GCNGRCVSGCAGRC und GCVLNGRMEC erhalten. Es konnte gezeigt werden, daß Fusionspolypeptide, welche diese Sequenzen und die Sequenz der ersten 218 Aminosäuren von humanem TF umfassen, in hohem Maße für die antivaskuläre Tumortherapie geeignet sind. Insbesondere konnte gezeigt werden, daß diese Fusionspolypeptide das Wachstum von Tumoren signifikant hemmen oder die Tumoren in Ihrer Größe reduzieren (sieh Fig. 7 und 8). Die beobachtete Induktion einer Teilremission der Tumoren (vgl. Fig. 8) verweist aufgrund der hohen Vorhersagekraft des Mausmodells (42, 43, 44) auf die zu erwartenden positiven Resultate bei der humanen Tumortherapie.

Weiterhin umfaßt die Erfindung auch Fusionsproteine mit zyklischen RGD-Peptiden, da durch die Zyklisierung die Affinität für Integrine verstärkt wird (wie beispielsweise in der Veröffentlichung 21 beschrieben).

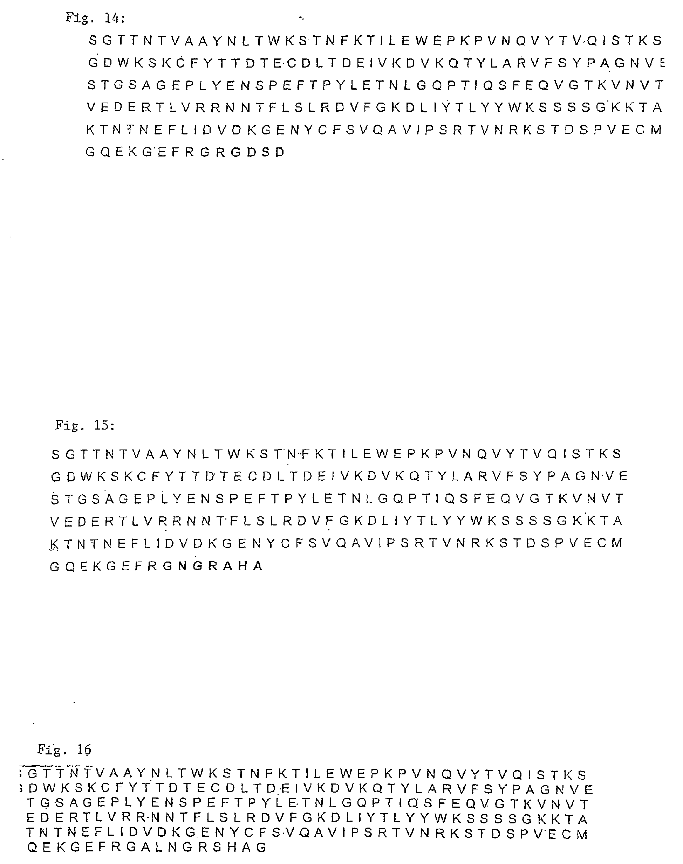

Die vorliegende Erfindung betrifft ferner Fusionspolypeptide, die eine der in SEQ ID NO:3-8 (Fig. 14-19) dargestellten Sequenzen aufweist.

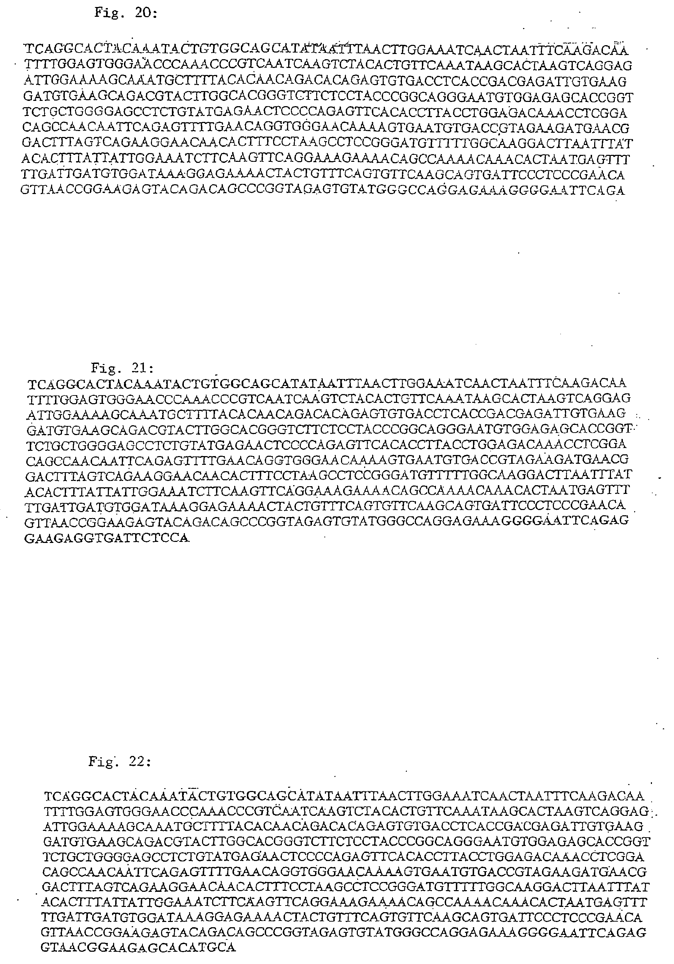

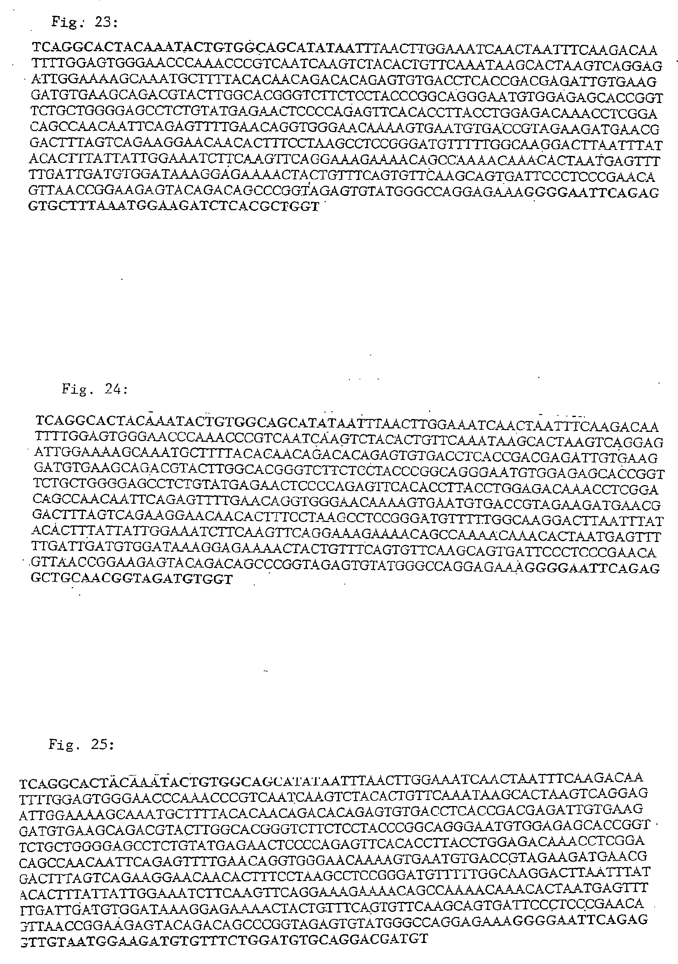

Gemäß einer weiteren Ausführungsform betrifft die vorliegende Erfindung Nukleinsäuren, die für ein Fusionspolypeptid, wie oben beschrieben, kodieren. Entsprechende Nukleinsäuren können beispielsweise eine der in SEQ ID NO: 10-15 (Fig. 21-26) dargestellten Sequenzen aufweisen.

In einem weiteren Aspekt betrifft die vorliegende Erfindung Vektoren, welche eine der oben genannten Nukleinsäuren umfassen. Entsprechende Vektoren umfassen üblicherweise ferner regulatorische Sequenzen für die Expression der Nukleinsäure. Solche Vektoren sind im Stand der Technik umfassend beschrieben und können von einer Vielzahl von Firmen kommerziell erworben werden.

In einer weiteren Ausführungsform betrifft die vorliegende Erfindung Zellen, welche eine der genannten Nukleinsäuren oder Vektoren enthalten. Die Zellen werden üblicherweise für die Expression der Nukleinsäure und die rekombinante Herstellung der erfindungsgemäßen Fusionspolypeptide verwendet. Für diesen Zweck können eine Vielzahl von Zellen zum Einsatz gelangen, darunter *E.coli*, Hefezellen und tierische Zelllinien, wie CHO- oder COS-Zellen. Entsprechende Zellen und deren Verwendung sind im Stand der Technik umfassend beschrieben.

Die erfindungsgemäßen Polypeptide nach Anspruch 1 können darüber hinaus auch durch andere geeignete Verfahren hergestellt werden, beispielsweise durch chemische Kopplung einzelner Peptide. So lassen sich einzelne Peptide nach im Stand der Technik üblichen Verfahren herstellen, z.B. durch chemische Synthese oder mittels heterologer Expression, und anschließend mittels Kopplung aneinanderfügen.

Schließlich betrifft die vorliegende Erfindung auch Arzneimittel, welche die oben beschriebenen Fusionspolypeptide, Nukleinsäuren, Vektoren oder Zellen umfassen. Die Arzneimittel können ferner pharmazeutisch verträgliche Träger, Hilfsmittel oder Adjuvanzien umfassen. Ferner können die Polypeptide in einem solchen Arzneimittel in einem modifizierten Zustand vorliegen, z.B. pegyliert, d.h. gekoppelt an ein Polyäthylenglykolmolekül.

Die erfindungsgemäßen Fusionspolypeptide bzw. diese Fusionspolypeptide enthaltende Arzneimittel werden für die Behandlung von Tumorerkrankungen, insbesondere für die antivaskuläre Tumortherapie verwendet. Als Tumorerkrankungen, die sich mit Hilfe der erfindungsgemäßen Fusionspolypeptide bzw. diese Fusionspolypeptide enthaltener Arzneimittel behandeln lassen, kommen beispielsweise Bronchialkarzinome und andere Tumoren des Thorax und des Mediastinums, Mammakarzinome und andere gynäkologische Tumoren, kolorektale Karzinome, Pankreaskarzinome und andere Tumoren des Gastrointestinaltraktes, maligne Melanome und andere Tumoren der Haut, Tumoren im Kopf-Hals-Bereich, Prostatakarzinome und andere Urogenitaltumoren, Sarkome, endokrin-aktive Tumoren, Leukämien und Myelodysplastische Syndrome sowie Hodgkin-Lymphome und Non-Hodgkin-Lymphome in Betracht.

Ferner können auch gutartige Tumoren, wie beispielsweise Hämangiome, und die Gefäßneubildung bei der diabetischen Retinopathie behandelt werden.

Neben der intravenösen Verabreichung ist auch die subkutane und intraperitonale Verabreichung der Fusionspolypeptide bzw. Arzneimittel möglich. Durch Verpackung in pharmazeutische Vehikel, die eine Spaltung der Fusionspolypeptide im Magen-Darm-Trakt verhindert, wäre es darüber hinaus auch möglich, die Fusionspolypeptide bzw. Arzneimittel oral zu verabreichen.

Vorteilhaft kann weiterhin eine Kombination der Verabreichung der erfindungsgemäßen Fusionspolypeptide mit anderen therapeutischen Ansätzen, z.B. einer cytotoxischen Chemotherapie oder Bestrahlung, sein. Auch eine Kombination mit anderen Wirkstoffen, z.B. eine Kombination mit Faktor Vlla oder Doxycyclin ist möglich, bevorzugt ist eine Kombination des erfindungsgemäßen Polypeptids mit Faktor VIIa oder Doxycyclin jedoch nicht notwendig.

Die Erfindung wird anhand der nachfolgenden Beispiele näher beschrieben:

### Beispiele

### Beispiel 1: Expression und Reinigung von tTF und tTF-Fusionsproteinen

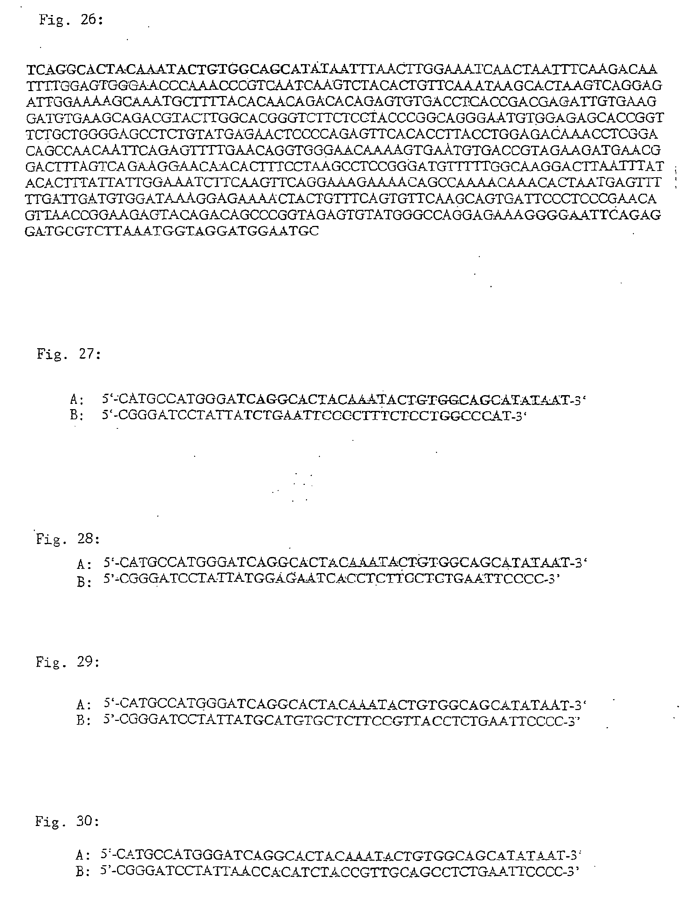

Die für die N-terminalen 218 Aminosäuren des Gewebefaktors TF (im folgenden als tTF bezeichnet) kodierende cDNA wurde mittels Polymerasekettenreaktion (PCR) unter Verwendung der in SEQ ID NO:16 und SEQ ID NO:17 (Fig. 27) synthetisiert und in den Expressionsvektor pET-30a(+) (Novagen) kloniert. Die rekombinanten Plasmide wurden in E. coli (BL21) transformiert, exprimiert und gereinigt (Qiagen Plasmid Kit).

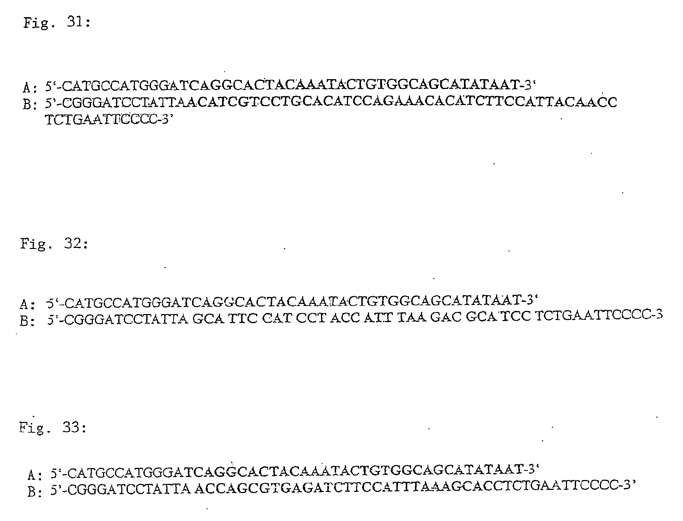

Neben dem trunkierten Gewebefaktor tTF wurden tTF-Peptid-Fusionsproteine konstruiert, in denen die Targeting-Peptide zunächst an das carboxyterminale Ende des löslichen Gewebefaktors tTF gebunden sind. Es wurden folgende lineare Fusionsproteine konstruiert:
**tTF-GRGDSP** (SEQ ID NO:3; Fig.14; im folgenden als tTF-RGD bezeichnet; es wurden die PCR-Primer SEQ ID NO:18 und SEQ ID NO:19 (Fig. 28) verwendet);
**tTF-GNGRAHA** (SEQ ID NO:4; Fig.15; im folgenden als tTF-NGR bezeichnet; es wurden die PCR-Primer SEQ ID NO:20 und SEQ ID NO:21 (Fig.29) verwendet);
**tTF-GALNGRSHAG** (SEQ ID NO:5; Fig.16; es wurden die PCR-Primer SEQ ID NO:28 und SEQ ID NO:29 (Fig. 33) verwendet);

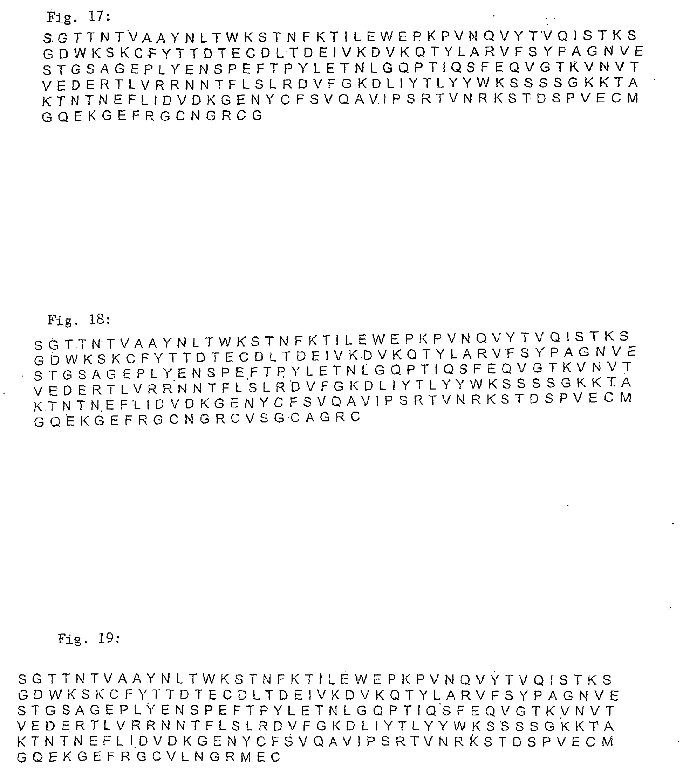

Darüber hinaus wurden die folgenden cyclischen Fusionsproteine synthetisiert:
**tTF-GCNGRCG** (SEQ ID NO:6; Fig.17; im folgenden als tTF-cycloNGR1 bezeichnet; es wurden die PCR-Primer SEQ ID NO:22 und SEQ ID NO:23 (Fig.30) verwendet),
**tTF-GCNGRCVSGCAGRC** (SEQ ID NO:7; Fig.18; im folgenden als tTF-cycloNGR2 bezeichnet; es wurden die PCR-Primer SEQ ID NO:24 und SEQ ID NO:25 (Fig.31) verwendet),
**tTF-GCVLNGRMEC** (SEQ ID NO:8; Fig.19; im folgenden als tTF-cycloNGR3 bezeichnet; es wurden die PCR-Primer SEQ ID NO:26 und SEQ ID NO:27 (Fig.32) verwendet)

Alle Konstrukte (einschließlich tTF) wurden in dem Vektor pET30a(+) exprimiert, der die zusätzliche Expression eines N-terminalen "Affinitäts-Tag" aus 6 Histidin-Resten und wenigen Vektorkodierten Aminosäuren vermittelt. Mit Hilfe dieses Affinitäts-Tags konnten die Konstrukte durch Affinitätschromatographie über eine Nickel-Nitrilotriessigsäure Säule (Ni-NTA, Novagen) gereinigt werden. Der Affinitäts-Tag ist in SEQ ID NO:30 dargestellt. SEQ ID NO:31 und SEQ ID NO:32 zeigen exemplarisch die vollständigen Aminosäuresequenzen von tTF-GRGDSP und tTF-GNGRAHA mit Affinitäts-Tag.

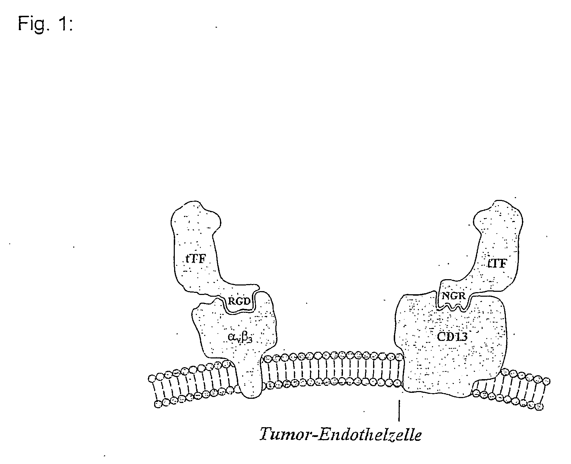

Die Konstrukte wurden so gewählt, daß auf der Basis der bekannten Röntgenkristallstruktur des tTF:FVIIa-Komplexes (19) eine senkrechte Ausrichtung des tTF-Fusionsproteins zur Phospholipidmembran der Endothelzelle gewährleistet wird, was der Ausrichtung des nativem TF entspricht. Andererseits wurde berücksichtigt, daß aus der gewählten Struktur keine sterische Behinderung von tTF für die Interaktion mit FVIIa und dem makromolekularen Substrat FX entstehen soll. Durch die Spezifität der RGD-Sequenz für das αᵥβ₃-Integrin und der NGR-Sequenz für CD13 (Aminopeptidase N) wird eine Tumorselektivität erreicht, da diese Rezeptoren selektiv und spezifisch in hoher Dichte auf Tumor-Endothelzellen, aber von wenigen Ausnahmen abgesehen, nicht auf ruhenden Endothelzellen im normalen Gewebe exprimiert werden (siehe Figur 1).

tTF und die beschriebenen Fusionsproteine tTF-RGD, tTF-NGR, tTF-GALNGRSHAG und tTF-cycloNGR1-3 wurden mittels pET30a(+) in E. coli (BL21) transformiert und exprimiert. Transformierte, mit IPTG induzierte *E*. *coli* BL21 DE3 wurden zentrifugiert und in 5-7 ml Lysispuffer (10 mM Tris-HCl, pH7,5; 150 mM NaCl; 1 mM MgCl₂; 10 µg/ml Aprotinin; 2 mg/ml Lysozym)/g Pellet aufgenommen und 20 µl Benzonase (Novagen) hinzugefügt. Nach 90 min Inkubation bei Raumtemperatur (RT) und einer Zentrifugation bei 12 000 g, 20 min, 4°C, wurde das Pellet resuspendiert und durch Ultraschallbehandlung in Waschpuffer (10 mM Tris/HCl, pH 7,5; 1 mM EDTA; 3% Triton X-100) homogenisiert. "Inclusion bodies" wurden über Nacht bei RT in 2-4 ml /g Pellet an denaturierendem Puffer (6 M Guanidiniumchlorid, 0,5 M NaCl, 20 mM NaH₂PO4, 1 mMDTT) gelöst. Der Überstand einer Zentrifugation (5 000 g, 30 min, 4°C) wurde mit einem 0,22 µg-Filter filtriert. Die Konstrukte wurden über eine Nickel-Nitrilotriessigsäure Säule (Ni-NTA, Novagen) über die zusätzlich eingeführten His-Tag Sequenzen des Konstrukts bis zur Homogenität gereinigt. Die Reinigung und Faltung der Proteine wurde mit dem His Bind Buffer Kit (Novagen) durchgeführt. Anschließend wurde gegen TBS-Puffer (20 mM Tris, 150 mM NaCl, pH 7,4) dialysiert.

Durch SDS-PAGE, Western Blot und Massenspektroskopie Analysen konnte die Identität der Proteine bestätigt werden (siehe Figur 2).

### Beispiel 2: Funktionelle Charakterisierung von tTF und tTF-Fusionsproteinen

Die funktionelle Aktivität dieser Fusionsproteine hinsichtlich Kofaktoraktivität bei der Aktivierung von Faktor X zu Faktor Xa durch Faktor Vlla konnte in vitro durch Michaelis-Menten Analysen gezeigt werden. Die Fähigkeit von tTF bzw. der tTF-Fusionspolypeptide, die spezifische proteolytische Aktivierung von FX durch FVIIa in Anwesenheit von Phospholipiden zu verstärken, wurde in einer leichten Modifikation nach der von Ruf beschriebenen Methode bestimmt (45). Hierzu wurden je 20 µl der folgenden Reagenzien in Mikrotiter-Platten pipettiert: (a) 50 nM rekombinanter FVIIa (Novo-Nordisk) in TBS-BSA; b) 0,16 nM - 1,6 µM tTF/tTF-Fusionspolypeptid in TBS-BSA; (c) 25 mM CaCl₂ und 500 µM Phospholipidvesikel (Phosphatidylcholin / Phosphatidylserin, 70/30, M/M; Sigma). Nach 10 Min. Inkubation bei Raumtemperatur wurden 20 µl des natürlichen Substrats FX (Enzyme Research Laboratories) in einer Konzentration von 5 µM hinzugegeben. Anschließend wurde eine Probe in minütlichen Abständen abpipettiert und die Reaktion durch Zugabe von 100 mM EDTA-Lösung gestoppt. Die gebildete Menge an FXa wurde durch Zugabe des chromogenen Substrats Spectrozym FXa in einem Microplate Reader durch Bestimmung der Absorptionsänderung bei 405 nm gemessen und die Parameter für die Michaelis-Menten-Kinetik nach der von Ruf angegebenen Methode analysiert. Die Ergebnisse zeigen, daß sowohl tTF als auch die tTF-Fusionspolypeptide unter diesen Bedingungen funktionell aktiv sind (Fig. 3). Die ermittelten Michaleliskonstanten (Km) der Fusionspolypeptide lagen zwischen 0,12 -1,2 nM (Fig. 3), somit im unteren Bereich, welcher für tTF publiziert wurde. Somit kann angenommen werden, daß durch die Fusionierung von tTF mit den Peptiden die funktionelle Aktivität unbeeinflusst bleibt.

### Beispiel 3: Bindung der tTF-Fusionsproteine an αᵥβ₃ in vitro und in vivo

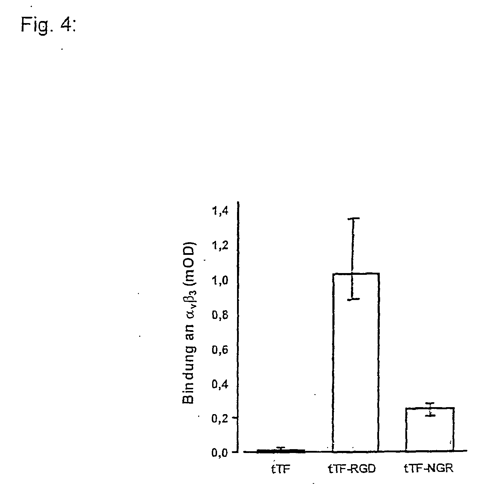

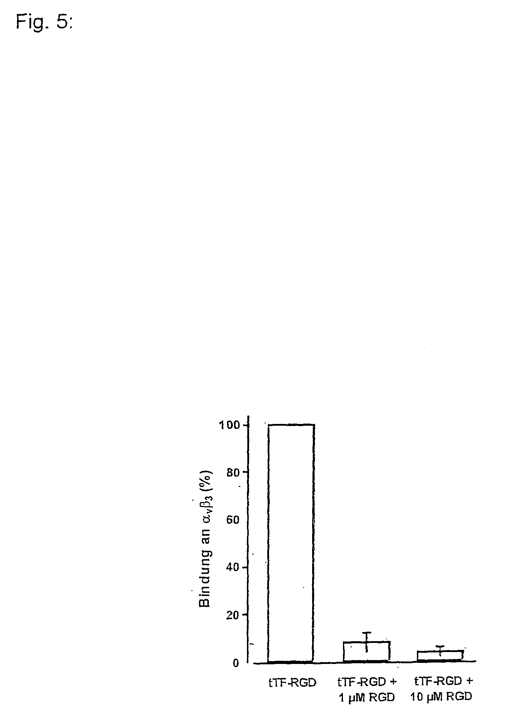

Die Bindung von tTF-RGD und tTF-NGR an das Integrin αᵥβ₃ konnte in einem ELISA (Enzyme Linked Immunosorbent Assay) demonstriert werden, indem gereinigtes αᵥβ₃ an Mikrotiterplatten immobilisiert worden war (siehe Figur 4). Die Spezifität der Bindung von tTF-RGD an αᵥβ₃ wird dadurch unterstrichen, daß das synthetische Peptid mit der Sequenz GRGDSP (Fa. Gibco) die Bindung von tTF-RGD an αᵥβ₃ in diesem Testsystem kompetitiv inhibiert (siehe Figur 5).

Anschließend wurde die spezifische Bindung von tTF-RGD an αᵥβ₃ auf Endothelzellen evaluiert. Hierzu wurde die differentielle Bindung von biotinyliertem tTF und tTF-RGD an Endothelzellen in Suspension mittels FACS (Fluorescence Activated Cell Sorting) analysiert. Hierbei macht man sich experimentell zu Nutze, daß alle in Gewebekultur gehaltenen Endothelzellen aktiviert sind, d.h. αᵥβ₃-Moleküle exprimieren. Dies läßt sich mittels verschiedener immunhistochemischer Verfahren nachweisen. Eine kultivierte Endothelzelle entspricht somit hinsichtlich ihres Expressionsmusters in Bezug auf αᵥβ₃ einer Tumorendothelzelle. Demgemäß läßt sich eine kultivierte Endothelzelle als Modellsystem für die spezifische Bindung von Substanzen an Tumorendothelzellen nutzen und ermöglicht ferner Aussagen über die zu erwartende Toxizität.

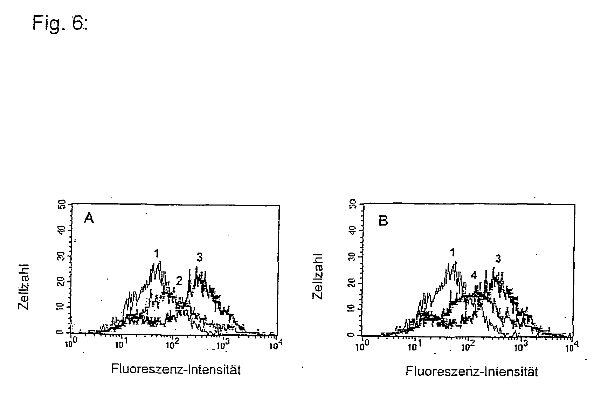

Als Detektionsmethode wurde Streptavidin-Phycoerythrin benutzt. Die gemessene Fluoreszenzintensität lag bei tTF-RGD um den Faktor acht höher als für tTF (Figur 6A). Ferner konnte die Bindung von 0,1 µM tTF-RGD an Endothelzellen kompetitiv durch die Gabe von 1 µM des synthetischen Peptids GRGDSP um 75% gesenkt werden (Figur 6B). Dies unterstreicht die Spezifität der Bindung von tTF-RGD an RGD-bindende Rezeptoren auf der Endothelzelloberfläche wie αᵥβ₃.

### Beispiel 4: Anti-Tumor Wirkung der tTF-Fusionsproteine im Tiermodell

Die Fusionsproteine tTF-RGD und tTF-NGR wurden hinsichtlich ihrer Wirkungen und Nebenwirkungen an Xenotransplantaten menschlicher Tumoren in thymuslosen Nacktmäusen evaluiert. Hierzu wurden die in unserem Labor etablierten Modelle verwendet (33, 34). Die Zellinien CCL185 (humanes Adenokarzinom der Lunge) bzw. M-21 (humanes Melanom) wurden subkutan in die Flanke männlicher BALB/c/Nackt-Mäuse (9-12 Wochen alt) injiziert. Nach Erreichen eines Tumorvolumens von etwa 50-100 mm³ (CCL185) bzw. 400-600 mm³ (M-21) wurden die Mäuse randomisiert vier Gruppen zugeordnet. Gruppe 1 erhielt nur physiologische Kochsalzlösung (NaCl), Gruppe 2 tTF, Gruppe 3 tTF-RGD, Gruppe 4 tTF-NGR (jeweils 1,5-2,0 mg/kg Körpergewicht (KG) des Proteins). Die Injektion erfolgte in die Schwanzvene der Tiere in 1-3 täglichen Intervallen (in Abhängigkeit von der Wachstumsgeschwindigkeit der jeweiligen Zellinie). Hier zeigte sich eine erhebliche therapeutische Aktivität der Fusionsproteine. Die Tumoren der mit tTF-RGD bzw. tTF-NGR Fusionsproteinen behandelten Mäuse wurden im Vergleich zu tTF oder NaCl in ihrem Wachstum signifikant gehemmt oder in ihrer Größe bis hin zu einer Teilremission reduziert (siehe Figuren 7 und 8).

Um den Wirkungsmechanismus der Thrombosierung von Tumorgefässen zu belegen, wurde folgendes Experiment durchgeführt: Hierzu wurde die humane Melanom Zellinie in die Flanke von zwei männlichen Balb/c/Nacktmäusen injiziert. Bei Erreichen einer Tumorgrösse von ca. 500 mm³ wurden 2,0 mg/kg KG tTF-NGR oder NaCl in die Schwanzvene injiziert. Figur 9A zeigt eine makroskopische in vivo Aufnahme der tumortragenden Maus 20 min. nach Injektion des tTF-NGR Fusionsproteins (linke Bildhälfte) bzw. NaCl (rechte Bildhälfte). Das makroskopische Bild mit bläulich-livider Verfärbung des Tumors nach Injektion von tTF-NGR deutet auf eine Tumornekrose hin. Nach 60 min. wurden die Mäuse exsanguiniert, der Tumor in toto exstipiert und histologisch untersucht. In Figur 9B ist die hämorrhagische Imbibierung des mit tTF-NGR behandelten Tumors als Zeichen der sekundären Einblutung infolge der beginnenden Tumornekrose sichtbar. Im Gegensatz hierzu scheint der mit NaCl behandelte Tumor vital zu sein (Figur 9C).

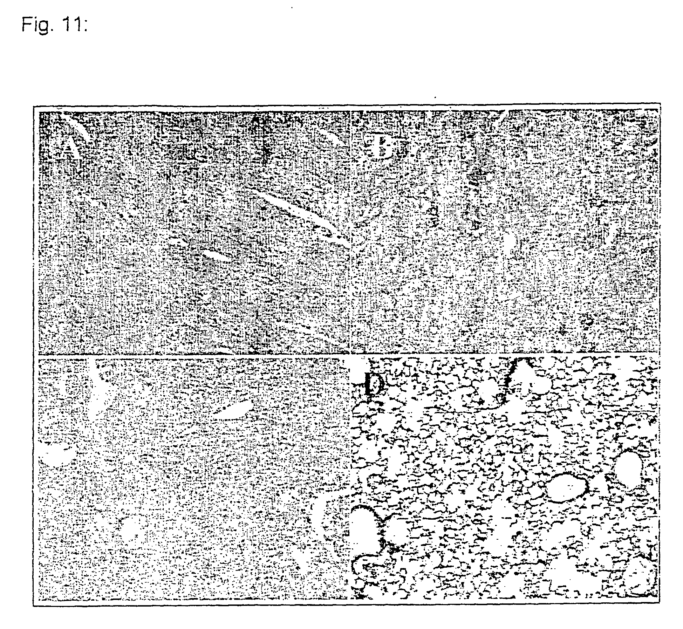

Die histologische Untersuchung des Melanom-Tumors zeigt mikroskopisch sichtbare Thrombenbildung in den Blutgefässen (Figur 10A-D). Durch diesen Befund wird der angenommene anti-Tumor-Wirkungsmechanismus von tTF-NGR, d.h. die Induktion von Thromben in den Blutgefässen, belegt. Die hohe Selektivität von tTF-NGR für Tumorblutgefässe wird durch den fehlenden histologischen Nachweis von Gerinnsel- und Nekrosenbildung im normalen Gewebe wie Herz, Niere, Leber und Lunge demonstriert (Figur 11A-D). Selbst repetitive hohe Dosen von tTF-NGR (4mg/kg KG) führten zu keinerlei sichtbarer Thrombenbildung oder Organtoxizität.

### Beispiel 5: Anti-Tumor Wirkung der tTF-Fusionsproteine im HT1080-Tumor-Tiermodell

Die Antitumoraktivität des tTF-RGD-Fusionsproteins wurde ferner in BALB/c/Nackt-Mäusen mit Fibrosarkomen (HT1080) untersucht. Diese Tumore wachsen schnell und sind gut vaskularisiert. The Ergebnisse zweier Experimente sind in Tabelle 2 und Figur 34 zusammengefaßt. Nach der zweiten Injektion von tTF-RGD wurde im Vergleich zu Kontollgruppen eine signifikante Wachstumsinhibition der HT1080 Tumore beobachtet. Dieser Effekt hielt bis zum Ende des Experiments an Tag 7 an (P=0,021 für tTF-RGD gegenüber der Puffer-Kontrolle (physiologische Kochsalzlösung), P=0,005 für tTF-RGD gegenüber tTF). Ähnlich wie bei den früheren Experimenten wurde eine Teilregression des Tumorvolumens in diesem Modell beobachtet.

**Tabelle 1:**

| Wirkung von tTF-RGD auf das Wachstum von M21-Tumoren in Mäusen | | | | | |
|---|---|---|---|---|---|
| Behandlung | Durchschnittliches Tumorvolumen (mm³) | | P ggü. Puffer | P ggü. tTF | n |
| | Tag 0 | Tag 7 | | | |
| Puffer | 590 +/-77 | 994 +/-140 | | ns | 9 |
| tTF | 558+/-47 | 931 +/-147 | ns | | 11 |
| tTF-RGD | 585+/-85 | 514+/-81 | <0,01 | <0,05 | 7 |

| | | | | | |
|---|---|---|---|---|---|
| ns: nicht signifikant | | | | | |

**Tabelle 2:**

| Wirkung von tTF-RGD auf das Wachstum von HT1080-Tumoren in Mäusen | | | | | |
|---|---|---|---|---|---|
| Behandlung | Durchschnittliches Tumorvolumen (mm³) | | P ggü. Puffer | P ggü. tTF | n |
| | Tag 0 | Tag 7 | | | |
| Puffer | 1671+/-296 | 2431+/-559 | | ns | 15 |
| tTF | 1751 +/-269 | 2335+/-398 | ns | | 14 |
| tTF-RGD | 1725+/-197 | 1241+/-122 | <0,05 | <0,01 | 12 |

| | | | | | |
|---|---|---|---|---|---|
| ns: nicht signifikant | | | | | |

**Tabelle 3:**

| Wirkung von tTF-RGD auf das Wachstum von CCL185-Tumoren in Mäusen | | | | | |
|---|---|---|---|---|---|
| Behandlung | Durchschnittliches Tumorvolumen (mm³) | | P ggü. Puffer | P ggü. tTF | n |
| | Tag 0 | Tag 7 | | | |
| Puffer | 39+/-3 | 467+/-137 | | ns | 9 |
| tTF | 44+/-8 | 764+/-148 | ns | | 5 |
| tTF-RGD | 45+/-5 | 130+/-19 | <0,01 | <0,01 | 10 |

| | | | | | |
|---|---|---|---|---|---|
| ns: nicht signifikant | | | | | |

Weitere tTF-Fusionsproteine können vom Fachmann auf der Basis der Offenbarung der vorliegenden Erfindung problemlos konstruiert werden. Potentielle Kandidaten sind die Peptide TAASGVRSMH und LTLRWVGLMS, welche an NG 2 binden, das murine Homolog des humanen Melanom Proteoglykans (12). Die Expression von NG 2 ist beschränkt auf Tumorzellen und angiogenen Gefäßen eines Tumors (35). Ein weiterer Kandidat ist das synthetische Peptid TTHWGFTL, welches selektiv und potent die Matrix-Metallo-Proteinase-2 (MMP-2) inhibiert (13). Da das Integrin αvβ3 offenbar auch die MMP-2 in einer RGD unabhängigen Weise bindet, wird hierdurch das aktive Enzym an die Oberfläche der angiogenen Blutgefäße lokalisiert (36). Ein Konstrukt bestehend aus tTF und diesem MMP-2 inhibitorischen Peptid könnte ebenfalls die selektive Bindung von tTF₁₋₂₁₈ an die Endothelzellmembran von Tumorgefäßen vermitteln.

### Literatur

1. Folkman J, Watson K, Ingber D, Hanahan D: Induction of angiogenesis during the transition from hyperplasia to neoplasia. Nature 339: 58-61, 1989
2. Dvrorak HJ, Sioussat TM, Brown LF, Berse B, Nagy JA, Sotrel A, Manseau EJ: Distribution of vascular permeability factor (vascular endothelial growth factor) in tumors - Concentration in tumor blood vessels. J Exp Med 174: 1275-1278, 1991
3. Dvorak HJ, Brown LF, Detmar M, Dvorak AM: Vascular permeability factor/vascular endothelial growth factor, microvascular hyperpermeability, and angiogenesis. Am J Pathol 146: 1029-1039, 1995
4. Terman BJ, Dougher-Vermazen M: Biological properties of VEGF/VPF receptors. Cancer Metastasis Rev 15: 159-163, 1996
5. Burrows FJ, Derbyshire EJ, Tazzari PL, Amlot P, Gazdzar AF, King SW, Letarte M, Vitetta ES, Thorpe PE: Upregulation of endoglin on vascular endothelial cells in human solid tumors: Implications for diagnosis and therapy. Clin Cancer Res 1: 1623-1634, 1995
6. Rettig WJ, Garinchesa P, Healey JH, Su SL, Jaffe EA, Old LJ: Identification of endosialin, a cell surface glycoprotein of vascular endothelial cells in human cancer. Proc Natl Acad Sci USA 89: 10832-10836, 1992
7. Carnemolla B, Balza E, Siri A, Zardi L, Nicrotra MR, Bigotti A, Natali PG: A tumor-associated fibronectin isoform generated by alternative splicing of messenger RNA precursors. J Cell Biol 108: 1139-1148, 1989
8. Arap W, Pasqualini R, Ruoslahti E: Cancer treatment by targeted drug delivery to tumor vasculature in a mouse model. Science 279: 377-380, 1998
9. Senger DR, Claffey KP, Benes JE, Peruzzi CA, Sergiou AP, Detmar M: Angiogenesis promoted by vascular endothelial growth factor: regulation through α1β1 and α2β1 integrins. Proc Natl Acad Sci USA 94: 13612-13617, 1997
10. Olson TA, Mohanraj D, Roy S, Ramakrishnan S: Targeting the tumor vasculature: inhibition of tumor growth by a vascular endothelial growth factor-toxin conjugate. Int J Cancer 73: 865-870, 1997
11. Bhagwat SV, Lahdenranta J, Giordano R, Arap W, Pasqualini R, Shapiro LH: CD13/APN is activated by angiogenic signals and is essential for capillary tube formation. Blood 97: 652-659, 2001
12. Burg MA, Pasqualini R, Arap W, Ruoslahti E, Stallcup WB: NG2 Proteoglycan-binding peptides target tumor neovasculature. Cancer Res 59: 2869-2874, 1999
13. Koivunen E, Arap W, Valtanen H, Rainisalo A, Medina OP, Heikkila P, Kantor C, Gahmberg CG, Salo T, Konttinen YT, Sorsa T, Ruoslahti E, Pasqualini R: Tumor targeting with a selective gelatinase inhibitor. Nat Biotechnol 17: 768-774, 1999
14. Huang X, Molema G, King S, Watkins L, Edgington TS, Thorpe PE: Tumor infarction in mice by antibody-directed targeting of tissue factor to tumor vasculature. Science 275: 547-550, 1997
15. Ran S, Gao B, Duffy S, Watkins L, Rote N, Thorpe PE: Infarction of solid Hodgkin's tumors in mice by antibody-directed targeting of tissue factor to tumor vasculature. Cancer Res 58: 4646-4653, 1998
16. Nilsson F, Kosmehl H, Zardi L, Neri D: Targeted delivery of tissue factor to the ED-B domain of fibronectin, a marker of angiogenesis, mediates the infarction of solid tumors in mice. Cancer Res 61: 711-716, 2001
17. Liu C, Huang H, Donate F, Dickinson C, Santucci R, EI-Sheikh A, Vessella R, Edgington TS. Prostate-specific membrane antigen directed selective thrombotic infarction of tumors. Cancer Res 62: 5470-5475, 2002
18. Gottstein C, Wels W, Ober B, Thorpe PE: Generation and characterisation of recombinant vascular targeting agents from hybridoma cell lines. BioTechniques 30: 190-200, 2001
19. Morrissey JH, Macik BG, Neuenschwander PF, Comp PC: Quantitation of activated factor VII levels in plasma using a tissue factor mutant selectively deficient in promoting factor VII activation. Blood 81: 734-744, 1993
20. Banner DW, D'Arcy A, Chène C, Winkler FK, Guha A, Konigsberg WH, Nemerson Y, Kirchhofer D: The crystal structure of the complex of blood coagulation factor Vlla with soluble tissue factor. Nature 380: 41-46, 1996
21. Koivunen E, Gay DA, Ruoslahti E: Selection of peptides binding to the α5β1 integrin from phage display library. J Biol Chem 268: 20205-20210, 1993
22. Healy JM, Murayama O, Maeda T, Yoshino K, Sekiguchi K, Kikuchi M: Peptide ligands for integrin αvβ3 selected from random phage display libraries. Biochemistry 34: 3948-3955, 1995
23. Pasqualini R, Koivunen E, Kain R, Lahdenranta J, Sakamoto M, Stryhn A, Ashmun RA, Shapiro LH, Arap W, Ruoslahti E: Aminopeptidase N is a receptor for tumor-homing peptides and a target for inhibiting angiogenesis. Cancer Res 60: 722-727, 2000
24. Curnis F, Sacchi A, Borgna L, Magni F, Gasparri A, Corti A: Enhancement of tumor necrosis factor α antitumor immunotherapeutic properties by targeted delivery to aminopeptidase N (CD 13). Nature Biotechnology 18: 1185-1190, 2000
25. Ellerby HM, Arap W, Ellerby LM, Kain R, Andrusiak R, Del Rio G, Krajewski S, Lombardo CR, Rao R, Ruoslahti E, Bredesen DE, Pasqualini R: Anti-cancer activity of targeted proapoptotic peptides. Nature Med 5: 1032-1038, 1999
26. Ruoslahti E: Targeting tumor vasculature with homing peptides from phage display. Cancer Biol 10:435-442,2000
27. Pasqualini R, Koivunen E, Kain R, Lahdenranta J, Sakamoto M, Stryhn A, Ashmun RA, Shapiro LH, Arap W, Ruohslahti E. Aminopeptidase N is a receptor for tumor-homing peptides and a traget for inhibiting angiogenesis. Cancer Res 60: 722-727, 2000
28. Curnis F, Arrigoni G, Sacchi A, Fischetti L, Arap W, Pasqualini R, Corti A. Differential binding of drugs containing the NGR motif to CD13 isoforms in tumor vessels, epithelia, and myeloid cells. Cancer Res 62: 867-874, 2002
29. Senger DR, Claffey KP, Benes JE, Perruzzi CA, Sergiou AP, Detmar M: Angiogenesis promoted by vascular endothelial growth factor: regulation through □1□1 and □2□1 integrins. Proc Natl Acad Sci USA 94: 13612-13617, 1997
30. Yun Z, Menter DG, Nicolson GL: Involvement of integrin αvβ3 in cell adhesion, motility and liver metastasis of murine RAW117 large cell lymphoma. Cancer Res 56: 3103-3111, 1996
31. Brooks PC, Clark RAF, Cheresh DA: Requirement of vascular integrin αvβ3 for angiogenesis. Science 264; 569-571, 1994
32. Brooks PC, Montgomery AM, Rosenfeld M, Reisfeld RA, Hu T, Klier G, Cheresh DA: Integrin αvβ3 antagonists promote tumor regression by inducing apoptosis of angiogenic blood vessels. Cell 92: 391-400, 1998
33. Topp MS, Koenigsmann M, Mire-Sluis A, Oberberg D, Eitelbach F, von Marschall Z, Notter M, Reufi B, Stein H, Thiel E, Berdel WE: Recombinant human interleukin-4 inhibits growth of some human lung tumor cell lines in vitro and in vivo. Blood 82: 2837-2844, 1993
34. Topp MS, Papadimitriou CA, Eitelbach F, Koenigsmann M, Oelmann E, Koehler B, Oberberg D, Reufi B, Stein H, Thiel E, Berdel WE: Recombinant human interleukin 4 has antiproliferative activity on human tumor cell lines derived from epithelial and non-epithelial histologies. Cancer Res 55: 2173-2176, 1995
35. Schrappe M, Klier FG, Spiro RC, Gladson CL: Correlation of chondroitin sulfate proteoglycan expression on proliferating brain capillary endothelial cells with the malignant phenotype of astroglial cells. Cancer Res 51: 4986-4993, 1991
36. Brooks PC: Localization of matrix metalloproteinase MMP-2 to the surface of invasive cells by interaction with integrin αvβ3. Cell 85: 683-693, 1996
37. Brooks PC, Silletti S, von Schalscha TL, Friedlander M, Cheresh DA: Disruption of Angiogenesis by PEX, a noncatalytic metalloproteinase fragment with integrin binding activity. Cell 92: 391-400, 1998
38. Schnurch H, Risau W: Expression of tie2, a member of a novel family of receptor tyrosine kinase in the endothelial cell lineage. Development 119: 957-968, 1993
39. Peters KG, Coogan A, Berry D, Marks J, Iglehart JD, Kontos CD, Rao P, Sankar S, Trogan E: Expression of tie2/tek in breast tumor vasculature provides a new marker for evaluation of tumor angiogenesis. Br J Cancer 77: 51-56, 1998
40. Suri C, Jones PF, Patan S, Bartunkova S, Maisonpierre PC, Davis S, Sato TN, Yancopoulos GD: requisite role of angiopoietin-1, a ligand for the tie2 receptor, during embryonic angiogenesis. Cell 87: 1171-1180, 1996
41. Maisonpierre PC, Suri C, Jones PF, Bartunkova S, Wiegand SJ, Radziejewski C, Compton D, McClain J, Aldrich TH, Papadopoulos N, Daly TH, Davis S, Sato TN, Yancopoulos GD:Angiopoietin-2, a natural antagonist for tie2 that disrupts in vivo angiogenesis. Science 277: 55-60, 1997
42. Scholz CC, Berger DP, Winterhalter BR, Henß H, Fiebig HH: Correlation of drug response in patients and in the clonogenic assay with solid human tumour xenografts. Eur J Cancer 26: 901-905, 1990
43. Fiebig HH, Berger DP, Dengler WA, Wallbrecher E, Winterhalter BR: Combined in vitro/in vivo test procedure with human tumor xenografts for new drug development. Contrib. Oncol. Basel, Karger 42: 321-351, 1992.
44. Fiebig HH, Burger AM: Human tumor xenografts and explants. Tumor Models in Cancer Research, eds B.A. Teicher, Humana Press Inc., Totowa, NJ, 2002.
45. Ruf W, Rehemtulla A, Edgington TS: Phospholipid-independent and -dependent Interactions required for tissue factor receptor and cofactor function. J Biol Chem 266: 2158-2166, 1991.
46. Hu P, Yan J, Sharifi J, Bai T, Khawla LA, Epstein AL: Comparison of three different targeted tissue factor fusion proteins for inducing tumor vessel thrombosis. Cancer Research 63: 5046-5053.
47. Rippmann JF, Pfizenmaier K, Mattes R, Rettig WJ, Moosmayer D: Fusion of the tissue factor extracellular domain to a tumour stroma specific single-chain fragment variable antibody results in an antigen-specific coagulation-promoting molecule. Biochem J. (2000) 349, 805-812.

## Patentansprüche

1. Fusionspolypeptid, umfassend
a) ein Peptid von 3 bis 30 Aminosäuren, das eine selektive Bindung des Fusionspolypeptides an Tumorgefäßendothelzellen ermöglicht; und
b) den Gewebefaktor TF (Tissue Factor) oder ein Fragment davon, wobei der Gewebefaktor und das Fragment **dadurch gekennzeichnet sind, daß** sie die Blutgerinnung bei Bindung des Fusionspolypeptides an Tumorgefäßendothelzellen aktivieren können,
wobei die Peptide a) und b) entweder unmittelbar oder über einen Linker mit bis zu 15 Aminosäuren aneinander gekoppelt sind, **dadurch gekennzeichnet, daß** das Peptid, das eine selektive Bindung des Fusionspolypeptides an Tumorgefäßendothelzellen ermöglicht, an den C-Terminus des Peptids, das die Blutgerinnung bei Bindung des Fusionspolypeptides an Tumorgefäßendothelzellen aktivieren kann, gekoppelt ist.

2. Fusionspolypeptid nach Anspruch 1, bestehend aus den Peptiden a) und b) und einem Linker mit bis zu 15 Aminosäuren.

3. Fusionspolypeptid nach Anspruch 1, wobei die Peptide a) und b) unmittelbar aneinander gekoppelt sind.

4. Fusionspolypeptid nach einem der Ansprüche 1 bis 3, **dadurch gekennzeichnet, daß** das Peptid, das die Blutgerinnung bei Bindung des Fusionspolypeptides an Tumorgefäßendothelzellen aktivieren kann, der Gewebefaktor TF ist, der die in SEQ ID NO:1 dargestellte Sequenz aufweist.

5. Fusionspolypeptid nach einem der Ansprüche 1 bis 3, **dadurch gekennzeichnet, daß** das Peptid, das die Blutgerinnung bei Bindung des Fusionspolypeptides an Tumorgefäßendothelzellen aktivieren kann, ein Fragment des Gewebefaktors TF ist, das vorzugsweise die in SEQ ID NO:2 dargestellte Sequenz aufweist.

6. Fusionspolypeptid nach einem der Ansprüche 1 bis 5, **dadurch gekennzeichnet, daß** das Peptid von 3 bis 30 Aminosäuren, das eine selektive Bindung des Fusionspolypeptides an Tumorgefäßendothelzellen ermöglicht, eine lineare oder cyclische Struktur aufweist.

7. Fusionspolypeptid nach einem der Ansprüche 1 bis 6, **dadurch gekennzeichnet, daß** das Peptid von 3 bis 30 Aminosäuren, das eine selektive Bindung des Fusionspolypeptides an Tumorgefäßendothelzellen ermöglicht, die Aminosäuresequenz RGD oder NGR umfaßt

8. Fusionspolypeptid nach Anspruch 7, **dadurch gekennzeichnet, daß** das Peptid, das eine selektive Bindung des Fusionspolypeptides an Tumorgefäßendothelzellen ermöglicht, aus der Gruppe bestehend aus GRGDSP und GNGRAHA ausgewählt wird.

9. Fusionspolypeptid nach Anspruch 7, **dadurch gekennzeichnet, daß** das Peptid, das eine selektive Bindung des Fusionspolypeptides an Tumorgefäßendothelzellen ermöglicht, aus der Gruppe bestehend aus GCNGRCG, GCNGRCVSGCAGRC, GCVLNGRMEC und GALNGRSHAG ausgewählt wird.

10. Fusionspolypeptid nach den Ansprüchen 1 bis 9, **dadurch gekennzeichnet, daß** es eine der in SEQ ID NO:3-8 dargestellten Sequenzen aufweist.

11. Nukleinsäure, die für ein Fusionspolypeptid nach einem der Ansprüche 1 bis 10 kodiert.

12. Nukleinsäure nach Anspruch 11, **dadurch gekennzeichnet, daß** sie eine der in SEQ ID NO:10-15 dargestellten Sequenzen aufweist.

13. Vektor, der eine Nukleinsäure nach Anspruch 11 oder 12 umfaßt.

14. Zelle, die eine Nukleinsäure nach Anspruch 11 oder 12 oder einen Vektor nach Anspruch 14 umfaßt.

15. Arzneimittel, das ein Fusionspolypeptid nach einem der Ansprüche 1 bis 10, eine Nukleinsäure nach Anspruch 11 oder 12, einen Vektor nach Anspruch 13 oder eine Zelle nach Anspruch 14 umfaßt.

16. Arzneimittel nach Anspruch 15, das ferner pharmazeutisch verträgliche Träger, Hilfsmittel oder Adjuvantien umfaßt.

## Claims

1. A fusion polypeptide, comprising
a) a peptide of 3 to 30 amino acids capable of selectively binding the fusion polypeptide to tumor vessel endothelial cells; and
b) a tissue factor (TF) or a fragment thereof, the tissue factor and the fragment being **characterized in that** they are able to activate blood clotting when the fusion polypeptide binds to tumor vessel endothelial cells,
wherein the peptides a) and b) are coupled to one another either directly or via a linker having up to 15 amino acids, **characterized in that** the peptide capable of selectively binding the fusion polypeptide to tumor vessel endothelial cells is coupled to the C-terminus of the peptide capable of activating blood clotting upon binding of the fusion polypeptide to tumor vessel endothelial cells.

2. The fusion polypeptide according to claim 1 consisting of the peptides a) and b) and a linker having up to 15 amino acids.

3. The fusion polypeptide according to claim 1 wherein the peptides a) and b) are coupled to one another directly.

4. The fusion polypeptide according to one of claims 1 to 3, **characterized in that** the peptide capable of activating blood clotting upon binding of the fusion polypeptide to tumor vessel endothelial cells is the tissue factor TF, which has the sequence shown in SEQ ID NO:1.

5. The fusion polypeptide according to one of claims 1 to 3, **characterized in that** the peptide capable of activating blood clotting upon binding of the fusion polypeptide to tumor vessel endothelial cells is a fragment of the tissue factor TF, which preferably has the sequence shown in SEQ ID NO:2.

6. The fusion polypeptide according to one of claims 1 to 5, **characterized in that** the peptide of 3 to 30 amino acids capable of selectively binding the fusion polypeptide to tumor vessel endothelial cells, has a linear or cyclic structure.

7. The fusion polypeptide according to one of claims 1 to 6, **characterized in that** the peptide of 3 to 30 amino acids capable of selectively binding the fusion polypeptide to tumor vessel endothelial cells comprises the amino acid sequence RGD or NGR.

8. The fusion polypeptide according to claim 7, **characterized in that** the peptide capable of selectively binding the fusion polypeptide to tumor vessel endothelial cells is selected from the group comprising GRGDSP and GNGRAHA.

9. The fusion polypeptide according to claim 7, **characterized in that** the peptide capable of selectively binding the fusion polypeptide to tumor vessel endothelial cells is selected from the group comprising GCNGRCG, GCNGRCVSGCAGRC, GCVLNGRMEC and GALNGRSHAG.

10. The fusion polypeptide according to claims 1 to 9, **characterized in that** it has one of the sequences shown in SEQ ID NO:3-8.

11. A nucleic acid encoding a fusion polypeptide according to one of claims 1 to 10.

12. The nucleic acid according to claim 11, **characterized in that** it has one of the sequences shown in SEQ ID NO:10-15.

13. A vector comprising a nucleic acid according to claim 11 or 12.

14. A cell comprising a nucleic acid according to claim 11 or 12 or a vector according to claim 14.

15. A pharmaceutical composition comprising a fusion polypeptide according to one of the claims 1 to 10, a nucleic acid according to claim 11 or 12, a vector according to claim 13 or a cell according to claim 14.

16. The pharmaceutical composition according to claim 15, which further comprises pharmaceutically acceptable carriers, excipients or adjuvants.

## Revendications

1. Polypeptide de fusion, comprenant
a) un peptide de 3 à 30 acides aminés, qui permet une liaison sélective du polypeptide de fusion à des cellules endothéliales vasculaires tumorales ; et
b) le facteur tissulaire TF (Tissue Factor) ou un fragment de ce dernier, dans lequel le facteur tissulaire et le fragment sont **caractérisés en ce qu'**ils peuvent activer la coagulation sanguine lors de la liaison du polypeptide de fusion à des cellules endothéliales vasculaires tumorales,
dans lequel les peptides a) et b) sont couplés l'un à l'autre, soit directement, soit via une séquence de liaison ayant jusqu'à 15 acides aminés, **caractérisé en ce que** le peptide qui permet une liaison sélective du polypeptide de fusion à des cellules endothéliales vasculaires tumorales est couplé à l'extrémité C-terminale du peptide, qui peut activer la coagulation sanguine lors de la liaison du polypeptide de fusion à des cellules endothéliales vasculaires tumorales.

2. Polypeptide de fusion selon la revendication 1, consistant en les peptides a) et b) et en une séquence de liaison ayant jusqu'à 15 acides aminés.

3. Polypeptide de fusion selon la revendication 1, dans lequel les peptides a) et b) sont directement couplés l'un à l'autre.

4. Polypeptide de fusion selon l'une des revendications 1 à 3, **caractérisé en ce que** le polypeptide qui peut activer la coagulation sanguine lors de la liaison du polypeptide de fusion à des cellules endothéliales vasculaires tumorales est le facteur tissulaire TF, qui présente la séquence représentée par SEQ ID NO:1.

5. Polypeptide de fusion selon l'une des revendications 1 à 3, **caractérisé en ce que** le peptide qui peut activer la coagulation sanguine lors de la liaison du polypeptide de fusion à des cellules endothéliales vasculaires tumorales est un fragment du facteur tissulaire TF, qui de préférence présente la séquence représentée par SEQ ID NO:2.

6. Polypeptide de fusion selon l'une des revendications 1 à 5, **caractérisé en ce que** le peptide de 3 à 30 acides aminés, qui permet une liaison sélective du polypeptide de fusion à des cellules endothéliales vasculaires tumorales, présente une structure linéaire ou cyclique.

7. Polypeptide de fusion selon l'une des revendications 1 à 6, **caractérisé en ce que** le peptide de 3 à 30 acides aminés, qui permet une liaison sélective du polypeptide de fusion à des cellules endothéliales vasculaires tumorales, comprend la séquence d'acides aminés RGD ou NGR.

8. Polypeptide de fusion selon la revendication 7, **caractérisé en ce que** le peptide qui permet une liaison sélective du polypeptide de fusion à des cellules endothéliales vasculaires tumorales est choisi dans le groupe consistant en GRGDSP et GNGRAHA.

9. Polypeptide de fusion selon la revendication 7, **caractérisé en ce que** le polypeptide qui permet une liaison sélective du polypeptide de fusion à des cellules endothéliales vasculaires tumorales est choisi dans le groupe consistant en GCNGRCG, GCNGRCVSGCAGRC, GCVLNGRMEC et GALNGRSHAG.

10. Polypeptide de fusion selon les revendications 1 à 9, **caractérisé en ce qu'**il présente l'une des séquences représentées par SEQ ID NO:3-8.

11. Acide nucléique qui code pour un polypeptide de fusion selon l'une des revendications 1 à 10.

12. Acide nucléique selon la revendication 11, **caractérisé en ce qu'**il présente l'une des séquences représentées par SEQ ID NO:10-15.

13. Vecteur qui comprend un acide nucléique selon la revendication 11 ou 12.

14. Cellule qui comprend un acide nucléique selon la revendication 11 ou 12 ou un vecteur selon la revendication 14.

15. Médicament qui comprend un polypeptide de fusion selon l'une des revendications 1 à 10, un acide nucléique selon la revendication 11 ou 12, un vecteur selon la revendication 13 ou une cellule selon la revendication 14.

16. Médicament selon la revendication 15 qui comprend en outre des supports, des agents auxiliaires ou des adjuvants pharmaceutiquement compatibles.
